# EUROPEAN PATENT APPLICATION

(11) **EP 3 647 323 A1**
(43) Date of publication of application: **06.05.2020**
(21) Application number: 18824945.2
(22) Date of filing: 29.06.2018
(51) Int. Cl.: C07K 16/28, C07K 16/46, C12N 15/13, A61K 39/395, A61P 35/00

(54) **ANTI-GITR ANTIBODY, ANTIGEN-BINDING FRAGMENT THEREOF, AND PHARMACEUTICAL USE THEREOF**

(30) Priority: 30.06.2017 CN 201710522742
(71) Applicant: Jiangsu Hengrui Medicine Co., Ltd., Jiangsu 222047 (CN); Shanghai Hengrui Pharmaceutical Co., Ltd, Shanghai 200245 (CN)
(72) Inventor: YAN, Shude, Lianyungang Jiangsu 222047 (CN); JIANG, Jiahua, Lianyungang Jiangsu 222047 (CN); HUANG, Hao, Lianyungang Jiangsu 222047 (CN); ZHANG, Lianshan, Lianyungang Jiangsu 222047 (CN); CAO, Guoqing, Lianyungang Jiangsu 222047 (CN)
(74) Representative: dompatent von Kreisler Selting Werner - Partnerschaft von Patent- und Rechtsanwälten mbB
(86) International application number: PCT/CN2018/093572
(87) International publication number: WO 2019/001559

(57) **Abstract**

Provided are an anti-GITR antibody, an antigen-binding fragment thereof, and pharmaceutical uses thereof. Further provided are a chimeric antibody and a humanized antibody containing a CDR of the anti-GITR antibody, a pharmaceutical composition containing the anti-GITR antibody or the antigen-binding fragment thereof, and use of the anti-GITR antibody in preparation of an anti-cancer drug. In particular, provided is a humanized anti-GITR antibody, and its use in the preparation of drugs for treating GITR-mediated diseases or disorders.

## Description

The present application claims the benefit of priority to Chinese patent application CN201710522742.4 filed on June 30, 2017, the entireties of which are incorporated herein by reference.

### Technical Field

The present invention relates to an anti-GITR antibody, an antigen-binding fragment of GITR, a chimeric antibody, a humanized antibody comprising a CDR region of the anti-GITR antibody, and a pharmaceutical composition comprising the human anti-GITR antibody or antigen-binding fragment thereof, and use of the anti-GITR antibody in the preparation of an anti-cancer drug.

### Background

As one of the top killers, tumors are a significant health challenge that human society will face for the forseeable future. Therapies such as traditional surgery, chemotherapy and radiotherapy often have a limited effect on the treatment of disseminated solid tumors. Tumor immunotherapy, especially T cell tumor immunotherapy, is a hot spot in the field of tumor therapy. Tumor immunotherapy makes the best use of cytotoxic T cells by mobilizing them in tumor patients to kill tumors, which may be the most effective and safest way to treat tumors. These therapies have shown promising prospects in the treatment of several different types of cancer, including disseminated metastatic tumors.

Activation of T cells in the human body employs a system comprising two signaling pathways. In additional delivery of a first signal to T cells by presenting MHC-antigenic peptide through antigen presenting cells (APC), a series of costimulatory molecules are required to provide a second signal, thus enabling the normal immune responses of T cells. This dual signaling pathway system plays a vital role in the balance of the immune system *in vivo.* It strictly regulates the body to activate different immune responses to self and non-self antigens. The absence of the second signal provided by the co-stimulator leads to failure of T cell responses or loss of sustained specific immune responses, thus resulting in immune tolerance. Therefore, the second signaling pathway plays a very critical role in regulating the whole process of an immune response.

GITR (glucocorticoid-induced TNFR-related protein, also known as TNFRSF18) is a transmembrane protein composed of 228 amino acids. It has a signal sequence of 19 amino acids, an extracellular region of 134 amino acids including three cysteine-rich motifs, a transmembrane region of 23 amino acids and an intracellular domain of 52 amino acids. The intracellular domain has high homology with the intracellular domains of TNFR, 4-1BB and CD27 in mice and humans. As a member of the tumor necrosis factor receptor (TNFR) superfamily, GITR is expressed in many components of the innate and adaptive immune system and has only minimal expression in a variety of normal tissues including breast, prostate, brain, heart, kidney, liver, salivary gland, spleen, stomach, thymus and uterus.

GITR is constitutively presented on inactive T cells and binds to another transmembrane protein, namely GITR ligand (GITRL). After activation of T cells, the expression of GITR increases, triggering co-activation of effector T lymphocytes (Teff) and regulating the activity of regulatory T cells (Treg). GITR is activated by the GITRL expressed mainly on APC, and the GITR transduces signals through its intracellular domain and triggers NF-kB activation via the TRAF2/NIK pathway. This activation process can increase resistance to tumors and viral infections and participate in autoimmune and inflammatory processes, including enhancing responses to infection and tumors through the co-activation of NK cells.

Currently a number of international companies are developing monoclonal antibodies against GITR, which improve patients' own immune system response to tumors and achieve the purpose of killing cancer cells through specifically stimulating immune activation. Related patent applications are WO2006105021, WO2011028683, WO2011028683, WO2013039954, WO2015184099, WO2015187835, WO2015031667, WO2016054638, WO2017087678 and WO2017068186 etc.. Up to now, the anti-GITR antibodies developed by Incyte, BMS and other companies are in phase I/IIa clinical trials, while related products owned by Novartis, Merck and other multinational pharmaceutical corporations are also in phase I clinical trials.

The present invention aims to provide anti-GITR antibodies with high affinity, high selectivity and high biological activity for use in drugs/compositions and methods that activate immune responses by stimulating GITR and its pathways.

### Summary of the Invention

The present invention provides an anti-GITR antibody or antigen-binding fragment thereof, comprising:
an antibody light chain variable region comprising at least one Light Chain Complementarity Determining Region (LCDR) selected from the group consisting of SEQ ID NO: 6, SEQ ID NO: 7, SEQ ID NO: 8; SEQ ID NO: 57, SEQ ID NO: 14, SEQ ID NO: 15, SEQ ID NO: 16; SEQ ID NO: 42, SEQ ID NO: 43, SEQ ID NO: 44; SEQ ID NO: 50, SEQ ID NO: 51 and SEQ ID: 52; and/or
an antibody heavy chain variable region comprising at least one Heavy Chain Complementarity Determining Region (HCDR) selected from the group consisting of SEQ ID NO: 3, SEQ ID NO: 4, SEQ ID NO: 5; SEQ ID NO: 11, SEQ ID NO: 12, SEQ ID NO: 56, SEQ ID NO: 13; SEQ ID NO: 39, SEQ ID NO: 40, SEQ ID NO: 41; SEQ ID NO: 47, SEQ ID NO: 48 and SEQ ID: 49.

In a preferred embodiment of the present invention, provided is an anti-GITR antibody or antigen-binding fragment thereof, as described above, wherein the antibody light chain variable region comprises an LCDR1 as shown in SEQ ID NO: 6, SEQ ID NO: 14, SEQ ID NO: 57, SEQ ID NO: 42 or SEQ ID NO: 50.

In a preferred embodiment of the present invention, provided is an anti-GITR antibody or antigen-binding fragment thereof, as described above, wherein the antibody light chain variable region comprises an LCDR2 as shown in SEQ ID NO: 7, SEQ ID NO: 15, SEQ ID NO: 43 or SEQ ID NO: 51.

In a preferred embodiment of the present invention, provided is an anti-GITR antibody or antigen-binding fragment thereof, as described above, wherein the antibody light chain variable region comprises an LCDR3 as shown in SEQ ID NO: 8, SEQ ID NO: 16, SEQ ID NO: 44 or SEQ ID NO: 52.

In a preferred embodiment of the present invention, provided is an anti-GITR antibody or antigen-binding fragment thereof, as described above, wherein the antibody heavy chain variable region comprises a HCDR1 as shown in SEQ ID NO: 3, SEQ ID NO: 11, SEQ ID NO: 39 or SEQ ID NO: 47.

In a preferred embodiment of the present invention, provided is an anti-GITR antibody or antigen-binding fragment thereof, as described above, wherein the antibody heavy chain variable region comprises a HCDR2 as shown in SEQ ID NO: 4, SEQ ID NO: 12, SEQ ID NO: 56, SEQ ID NO: 40 or SEQ ID NO: 48.

In a preferred embodiment of the present invention, provided is an anti-GITR antibody or antigen-binding fragment thereof, as described above, wherein the antibody heavy chain variable region comprises a HCDR3 as shown in SEQ ID NO: 5, SEQ ID NO: 13, SEQ ID NO: 41 or SEQ ID NO: 49.

In a preferred embodiment of the present invention, provided is an anti-GITR antibody or antigen-binding fragment thereof, as described above, wherein the antibody light chain variable region comprises an LCDR1, LCDR2 and LCDR3 as shown in SEQ ID NO: 6, SEQ ID NO: 7 and SEQ ID NO: 8, respectively.

In a preferred embodiment of the present invention, provided is an anti-GITR antibody or antigen-binding fragment thereof, as described above, wherein the antibody light chain variable region comprises an LCDR1, LCDR2 and LCDR3 as shown in SEQ ID NO: 57, SEQ ID NO: 15 and SEQ ID NO: 16, respectively.

In a preferred embodiment of the present invention, provided is an anti-GITR antibody or antigen-binding fragment thereof, as described above, wherein the antibody light chain variable region comprises an LCDR1, LCDR2 and LCDR3 as shown in SEQ ID NO: 14, SEQ ID NO: 15 and SEQ ID NO: 16, respectively.

In a preferred embodiment of the present invention, provided is an anti-GITR antibody or antigen-binding fragment thereof, as described above, wherein the antibody light chain variable region comprises an LCDR1, LCDR2 and LCDR3 as shown in SEQ ID NO: 42, SEQ ID NO: 43 and SEQ ID NO: 44, respectively.

In a preferred embodiment of the present invention, provided is an anti-GITR antibody or antigen-binding fragment thereof, as described above, wherein the antibody light chain variable region comprises an LCDR1, LCDR2 and LCDR3 as shown in SEQ ID NO: 50, SEQ ID NO: 51 and SEQ ID NO: 52, respectively.

In a preferred embodiment of the present invention, provided is an anti-GITR antibody or antigen-binding fragment thereof, as described above, wherein the antibody heavy chain variable region comprises a HCDR1, HCDR2 and HCDR3 as shown in SEQ ID NO: 3, SEQ ID NO: 4 and SEQ ID NO: 5, respectively.

In a preferred embodiment of the present invention, provided is an anti-GITR antibody or antigen-binding fragment thereof, as described above, wherein the antibody heavy chain variable region comprises a HCDR1, HCDR2 and HCDR3 as shown in SEQ ID NO: 11, SEQ ID NO: 56 and SEQ ID NO: 13, respectively.

In a preferred embodiment of the present invention, provided is an anti-GITR antibody or antigen-binding fragment thereof, as described above, wherein the antibody heavy chain variable region comprises an HCDR1, HCDR2 and HCDR3 as shown in SEQ ID NO: 11, SEQ ID NO: 12 and SEQ ID NO: 13, respectively.

In a preferred embodiment of the present invention, provided is an anti-GITR antibody or antigen-binding fragment thereof, as described above, wherein the antibody heavy chain variable region comprises a HCDR1, HCDR2 and HCDR3 as shown in SEQ ID NO: 39, SEQ ID NO: 40 and SEQ ID NO: 41, respectively.

In a preferred embodiment of the present invention, provided is an anti-GITR antibody or antigen-binding fragment thereof, as described above, wherein the antibody heavy chain variable region comprises a HCDR1, HCDR2 and HCDR3 as shown in SEQ ID NO: 47, SEQ ID NO: 48 and SEQ ID NO: 49, respectively.

In a preferred embodiment of the present invention, provided is an anti-GITR antibody or antigen-binding fragment thereof, as described above, wherein the antibody light chain variable region comprises the LCDR1, LCDR2 and LCDR3 as shown in SEQ ID NO: 6, SEQ ID NO: 7 and SEQ ID NO: 8, respectively; or the LCDR1, LCDR2 and LCDR3 as shown in SEQ ID NO: 57, SEQ ID NO: 15 and SEQ ID NO: 16, respectively; or the LCDR1, LCDR2 and LCDR3 as shown in SEQ ID NO: 14, SEQ ID NO: 15 and SEQ ID NO: 16, respectively; or the LCDR1, LCDR2 and LCDR3 as shown in SEQ ID NO: 42, SEQ ID NO: 43 and SEQ ID NO: 44, respectively; or the LCDR1, LCDR2 and LCDR3 as shown in SEQ ID NO: 50, SEQ ID NO: 51 and SEQ ID NO: 52, respectively;
wherein the antibody heavy chain variable region comprises the HCDR1, HCDR2 and HCDR3 as shown in SEQ ID NO: 3, SEQ ID NO: 4 and SEQ ID NO: 5, respectively; or the HCDR1, HCDR2 and HCDR3 as shown in SEQ ID NO: 11, SEQ ID NO: 56 and SEQ ID NO: 13, respectively; or the HCDR1, HCDR2 and HCDR3 as shown in SEQ ID NO: 11, SEQ ID NO: 12 and SEQ ID NO: 13, respectively; or the HCDR1, HCDR2 and HCDR3 as shown in SEQ ID NO: 39, SEQ ID NO: 40 and SEQ ID NO: 41, respectively; or the HCDR1, HCDR2 and HCDR3 as shown in SEQ ID NO: 47, SEQ ID NO: 48 and SEQ ID NO: 49, respectively.

Particularly preferably, the anti-GITR antibody or antigen-binding fragment thereof may be any one selected from of the group consisting of:
(1) the antibody light chain variable region comprising the LCDR1, LCDR2 and LCDR3 as shown in SEQ ID NO: 6, SEQ ID NO: 7 and SEQ ID NO: 8, respectively; and the heavy chain variable region comprising the HCDR1, HCDR2 and HCDR3 as shown in SEQ ID NO: 3, SEQ ID NO: 4 and SEQ ID NO: 5, respectively;
(2) the antibody light chain variable region comprising the LCDR1, LCDR2 and LCDR3 as shown in SEQ ID NO: 57, SEQ ID NO: 15 and SEQ ID NO: 16, respectively; and the heavy chain variable region comprising the HCDR1, HCDR2 and HCDR3 as shown in SEQ ID NO: 11, SEQ ID NO: 56 and SEQ ID NO: 13, respectively;
(3) the antibody light chain variable region comprising the LCDR1, LCDR2 and LCDR3 as shown in SEQ ID NO: 14, SEQ ID NO: 15 and SEQ ID NO: 16, respectively; and the heavy chain variable region comprising the HCDR1, HCDR2 and HCDR3 as shown in SEQ ID NO: 11, SEQ ID NO: 12 and SEQ ID NO: 13, respectively;
(4) the antibody light chain variable region comprising the LCDR1, LCDR2 and LCDR3 as shown in SEQ ID NO: 42, SEQ ID NO: 43 and SEQ ID NO: 44, respectively; and the heavy chain variable region comprising the HCDR1, HCDR2 and HCDR3 as shown in SEQ ID NO: 39, SEQ ID NO: 40 and SEQ ID NO: 41, respectively; and
(5) the antibody light chain variable region comprising the LCDR1, LCDR2 and LCDR3 as shown in SEQ ID NO: 50, SEQ ID NO: 51 and SEQ ID NO: 52, respectively; and the heavy chain variable region comprising the HCDR1, HCDR2 and HCDR3 as shown in SEQ ID NO: 47, SEQ ID NO: 48 and SEQ ID NO: 49, respectively.

In a preferred embodiment of the present invention, the sequence of the antibody light chain variable region is selected from SEQ ID NO: 2 and SEQ ID NO: 10; the sequence of the antibody heavy chain variable region is selected from SEQ ID NO: 1 and SEQ ID NO: 9.

The present invention also provides an anti-GITR antibody or antigen-binding fragment thereof, wherein the light chain variable region, LCVR, of the antibody or antigen-binding fragment has a sequence of SEQ ID NO: 38 and the heavy chain variable region, HCVR, has a sequence of SEQ ID NO: 37.

The present invention also provides an anti-GITR antibody or antigen-binding fragment thereof, wherein the light chain variable region, LCVR, of the antibody or antigen fragment has a sequence of SEQ ID NO: 46 and the heavy chain variable region, HCVR, has a sequence of SEQ ID NO: 45.

In a preferred embodiment of the present invention, provided is an anti-GITR antibody or antigen-binding fragment thereof, as described above, wherein the antibody is a murine antibody or a fragment thereof.

In a preferred embodiment of the present invention, provided is a murine antibody or a fragment thereof, as described above, wherein the antibody light chain variable region further comprises a light chain FR region of murine κ, λ chain or variants thereof.

In a preferred embodiment of the present invention, provided is a murine antibody or a fragment thereof, as described above, wherein the antibody light chain variable region further comprises a light chain constant region of murine κ, λ chain or variants thereof.

In a preferred embodiment of the present invention, provided is a murine antibody or a fragment thereof, as described above, wherein the antibody heavy chain variable region further comprises a heavy chain FR region of murine IgG1, IgG2, IgG3, IgG4 or variants thereof.

In a preferred embodiment of the present invention, provided is a murine antibody or a fragment thereof, as described above, wherein the antibody light chain variable region further comprises a heavy chain constant region of murine IgG1, IgG2, IgG3, IgG4 or variants thereof.

In a preferred embodiment of the present invention, provided is an anti-GITR antibody or antigen-binding fragment thereof, as described above, wherein the antibody is a chimeric antibody or fragment thereof.

In a preferred embodiment of the present invention, provided is a murine antibody or chimeric antibody, as described above, wherein the antibody light chain variable region, LCVR, has a sequence of SEQ ID NO: 2 or SEQ ID NO: 10.

In a preferred embodiment of the present invention, provided is a murine antibody or chimeric antibody, as described above, wherein the antibody heavy chain variable region, HCVR, has a sequence of SEQ ID NO: 1 or SEQ ID NO: 9.

In a preferred embodiment of the present invention, provided is a chimeric antibody or a fragment thereof, as described above, further comprising a light chain constant region of human κ, λ chain or variants thereof.

In a preferred embodiment of the present invention, provided is a chimeric antibody or a fragment thereof, as described above, further comprising a heavy chain constant region of human IgG1, IgG2, IgG3, IgG4 or variants thereof.

In a preferred embodiment of the present invention, provided is an anti-GITR antibody or antigen-binding fragment thereof, as described above, wherein the antibody is a human antibody or fragment thereof.

In a preferred embodiment of the present invention, provided is an anti-GITR antibody or antigen-binding fragment thereof, as described above, wherein the antibody is a humanized antibody or a human antibody.

In a preferred implementation of the present invention, provided is a humanized antibody, as described above, having a antibody light chain sequence of SEQ ID NO: 18 or SEQ ID NO: 20 or variants thereof; the variant preferably has 0-10 amino acid mutations in the light chain, and more preferably has mutations at amino acid positions 46, 49, 52 and 57; wherein the amino acid sequence of SEQ ID NO: 18 is preferably mutated at position 49, and the mutation of the amino acid is preferably S49P; wherein the amino acid sequence of SEQ ID NO: 20 is preferably mutated at positions 46, 52 and 57, and the mutations of the amino acids are preferably W46L, D52N and F57I.

In a preferred embodiment of the present invention, provided is a humanized antibody, as described above, wherein the antibody heavy chain variable region has a sequence of SEQ ID NO: 17 or SEQ ID NO: 19 or variants thereof; the variant preferably has 0-10 amino acid mutations in the light chain.

In a preferred embodiment of the present invention, provided is a human antibody or fragment thereof, as described above, further comprising the constant region of human IgG1, IgG2, IgG3, IgG4 or variants thereof, preferably comprising the constant region of human IgG1, IgG2 or IgG4.

In a preferred embodiment of the present invention, provided is an anti-GITR antibody or antigen-binding fragment thereof, as described above, wherein the antibody is a humanized antibody or fragment thereof.

In a preferred embodiment of the present invention, provided is a humanized antibody or fragment thereof, as described above, wherein the humanized antibody light chain variable region further comprises a light chain FR region of human κ, λ chain or variants thereof.

In a preferred embodiment of the present invention, provided is a humanized antibody or fragment thereof, as described above, wherein the sequence of the light chain FR region of the humanized antibody light chain variable region is derived from the light chain IGkV4-1 sequence of the human germline as shown in SEQ ID NO: 22, or the light chain IGkV3-11 sequence of the human germline as shown in SEQ ID NO: 24.

In a preferred embodiment of the present invention, provided is a humanized antibody or fragment thereof, as described above, wherein the humanized antibody light chain variable region is shown as SEQ ID NO: 29 or SEQ ID NO: 36, or variants thereof.

In a preferred embodiment of the present invention, provided is a humanized antibody or fragment thereof, as described above, further comprising a light chain constant region of human κ, λ chain or variants thereof.

In a preferred embodiment of the present invention, provided is a humanized antibody or fragment thereof, as described above, wherein the humanized antibody heavy chain variable region further comprises a heavy chain FR region of human IgG1, IgG2, IgG3, IgG4 or variants thereof.

In a preferred embodiment of the present invention, provided is a humanized antibody or fragment thereof, as described above, wherein the sequence of the heavy chain FR region of the humanized antibody heavy chain variable region is derived from the heavy chain IGHV3-38 sequence of the human germline as shown in SEQ ID NO: 21, or the heavy chain IGHV1-2 sequence of the human germline as shown in SEQ ID NO: 23.

In a preferred embodiment of the present invention, provided is a humanized antibody or fragment thereof, as described above, wherein the humanized antibody heavy chain variable region is shown as SEQ ID NO: 26 or SEQ ID NO: 33, or variants thereof.

In a preferred implementation of the present invention, provided is a humanized antibody or fragment thereof, as described above, wherein the humanized antibody heavy chain sequence is shown as SEQ ID NO: 17 or SEQ ID NO: 19 or variants thereof; the variant preferably has 0-10 amino acid mutations in the heavy chain, and more preferably has mutations at amino acid positions 46, 61 and 76; wherein the amino acid sequence of SEQ ID NO: 17 is preferably mutated at position 49, and the mutation of amino acid is preferably A49S; wherein the amino acid sequence of SEQ ID NO: 19 is preferably mutated at positions 61 and 76, and the mutations of the amino acids are preferably N61A and S76I.

In a preferred embodiment of the present invention, provided is a humanized antibody or fragment thereof, as described above, further comprising a heavy chain constant region of human IgG1, IgG2, IgG3, IgG4 or variants thereof, preferably comprising the heavy chain FR region of human IgG1, IgG2 or IgG4, more preferably comprising the heavy chain FR region of human IgG1 or IgG2.

In a preferred embodiment of the present invention, provided is an anti-GITR antibody or antigen-binding fragment thereof, as described above, wherein the antigen-binding fragment is a Fab, a Fv, a sFv, a F(ab')2, a linear antibody, a single chain antibody, a nanobody, a domain antibody or a multispecific antibody.

The present invention further provides an isolated monoclonal antibody or antigen-binding fragment thereof, which can compete with the above monoclonal antibody or antigen-binding fragment thereof for binding to GITR.

The present invention further provides a nucleic acid encoding the anti-GITR antibody or antigen-binding fragment thereof.

The present invention further provides an expression vector comprising the nucleic acid, as described above.

The present invention further provides a host cell transformed with the expression vector, as described above.

In a preferred embodiment of the present invention, provided is a host cell, as described above, wherein the host cell is a bacterium, preferably *Escherichia coli.*

In a preferred embodiment of the present invention, provided is a host cell, as described above, wherein the host cell is a yeast, preferably *Pichia pastoris.*

In a preferred embodiment of the present invention, provided is a host cell, as described above, wherein the host cell is a mammalian cell, preferably a Chinese hamster ovary (CHO) cell or a human embryonic kidney (HEK) 293 cell.

The present invention also provides a multi-specific antibody comprising the light chain variable region and the heavy chain variable region, as described above.

The present invention also provides a single chain antibody comprising the light chain variable region and the heavy chain variable region, as described above.

The present invention also provides an antibody-drug conjugate comprising the light chain variable region and/or the heavy chain variable region, as described above. Antibody-drug conjugates are known in the art to be formed by antibody-linker-drug (toxin) interconnected with each other. Linkers known in the art comprise cleavable linkers, uncleavable linkers, e.g. the linkers comprise but are not limited to SMCC, SPDP, etc.; toxins are also known in the art, such as DM1, DM4, MMAE, MMAF, etc.

The present invention also provides a method for preparing an anti-GITR antibody and antigen-binding fragment thereof, comprising expressing the antibody or antigen-binding fragment thereof in the host cell, as described above, and isolating the antibody or antigen-binding fragment thereof from the host cell.

The present invention further provides a pharmaceutical composition comprising the anti-GITR antibody or antigen-binding fragment thereof, the isolated monoclonal antibody or antigen-binding fragment thereof, the multi-specific antibody or the single chain antibody, as described above, and a medicinal excipient, diluent or carrier.

The present invention further provides use of the anti-GITR antibody or antigen-binding fragment thereof, the isolated monoclonal antibody or antigen-binding fragment thereof, the multi-specific antibody, the single chain antibody or the pharmaceutical composition, as described above, in the preparation of a drug for treating GITR- or GITRL-mediated diseases or disorders; wherein the disease is preferably cancer; the cancer is most preferably melanoma, breast cancer, ovarian cancer, prostate cancer, pancreatic cancer, renal cancer, lung cancer, liver cancer, gastric cancer, colorectal cancer, bladder cancer, esophageal cancer, cervical cancer, multiple myeloma, leukemia, lymphoma, gallbladder cancer or glioblastoma.

The present invention further provides a method for treating and preventing GITR- or GITRL-mediated diseases or disorders, comprising administering to the patient in need thereof a therapeutically effective amount of the anti-GITR antibody or antigen-binding fragment thereof, the isolated monoclonal antibody or antigen-binding fragment thereof, the multi-specific antibody, the single chain antibody or the pharmaceutical composition, as described above; wherein the disease is preferably cancer; the cancer is most preferably melanoma, breast cancer, ovarian cancer, prostate cancer, pancreatic cancer, renal cancer, lung cancer, liver cancer, gastric cancer, colorectal cancer, bladder cancer, esophageal cancer, cervical cancer, multiple myeloma, leukemia, lymphoma, gallbladder cancer or glioblastoma.

### Description of the Drawings

Fig. 1 shows cellular activity of murine anti-GITR antibody *in vitro,* showing that each tested antibody can effectively stimulate the secretion of cytokine IL-2, and the function of each antibody was assessed by the concentration of stimulated IL-2.
Fig. 2 shows cellular activity of humanized anti-GITR antibody *in vitro,* showing that the tested antibodies hu18F10 and hu8A11 can both effectively stimulate the secretion of cytokine IL-2, and their function is comparable to or greater than that of the control antibody BMAB.
Fig. 3 shows a tumor growth curve of the NCG mice having A375 melanoma co-transplanted with human PBMC.
Fig. 4 shows weight change curve of the NCG mice having A375 melanoma co-transplanted with human PBMC.

### Detailed Description of the Invention

### 1. Definitions

Some technical and scientific terms are defined as follows for the better understanding of the present invention. Unless defined otherwise, all technical and scientific terms used herein have the same meaning as is commonly understood by one of skill in the art to which this invention belongs.

The amino acid three-letter code and single-letter code used in the present invention are described in J. Biol. Chem, 243, p3558 (1968).

The term "antibody" of the present invention refers to an immunoglobulin, which is a tetrapeptide chain structure consisting of two identical heavy chains and two identical light chains linked by interchain disulfide bonds. The composition and sequence of amino acids in the constant region of immunoglobulin heavy chains are different, so their antigenicity is also different. Accordingly, immunoglobulins can be classified into five classes, or isotypes of immunoglobulins, namely IgM, IgD, IgG IgA and IgE, and the corresponding heavy chains of these isotypes are µ chain, δ chain, γ chain, α chain and ε chain, respectively. According to the difference in amino acid composition of the hinge region and the number and position of heavy chain disulfide bonds, the same type of IgGs can be further divided into different subclasses, such as IgG 1, IgG 2, IgG 3 and IgG 4. Light chains can be classified as κ chains or λ chains according to differences in the constant regions. Each of the five classes of Ig may have a κ chain or a λ chain.

In the present invention, the antibody light chain of the present invention can further comprise a light chain constant region, which includes a human or murine κ, λ chain or variants thereof.

In the present invention, the antibody heavy chain of the present invention can further comprise a heavy chain constant region, which includes a human or murine IgG1, 2, 3, 4 or variants thereof.

The variable region (V region), comprises about 110 amino acids close to the N-terminus of the antibody heavy and light chains, varies considerably in its amino acid sequence. The constant region (C region), comprises the remaining amino acid sequence close to the C-terminus of the antibody, is relatively stable. The variable region comprises three hypervariable regions (HVR) and four relatively conserved framework regions (FR). The three hypervariable regions, which determine the specificity of the antibody, are also termed complementarity determining regions (CDRs). Each of the light chain variable region (VL) and the heavy chain variable region (VH) consists of three CDR regions and four FR regions, arranged from amino terminus to carboxy terminus in the following order: FR1, CDR1, FR2, CDR2, FR3, CDR3 and FR4. The three CDR regions of the light chain refer to LCDR1, LCDR2, and LCDR3; the three CDR regions of the heavy chain refer to HCDR1, HCDR2, and HCDR3. The number and position of CDR amino acid residues of the VL and VH regions of the antibodies or antigen-binding fragments of the invention comply with known IMGT numbering criteria.

The term "recombinant human antibody" includes a human antibody prepared, expressed, created or isolated by a recombinant method. The related techniques and methods are well known in the art, such as (1) an antibody is isolated from a transgenic or transchromosomic animal (such as a mouse) containing a human immunoglobulin gene, or an antibody is isolated from the hybridoma prepared therefrom, (2) an antibody is isolated from a host cell that has been transformed to express an antibody, such as a transfected tumor, (3) an antibody is isolated from a recombinant combined human antibody library, and (4) an antibody is prepared, expressed, created or isolated by splicing human immunoglobulin gene sequences into other DNA sequences and the like. These recombinant human antibodies comprise not only variable and constant regions that utilize specific human germline immunoglobulin sequences encoded by germline genes, but also subsequent rearrangements and mutations such as those occur during the maturation of an antibody.

The term "murine antibody" of the present invention is a monoclonal antibody against human GITR prepared according to the knowledge and skills in the art. GITR antigen is injected into the test subjects during preparation, and hybridomas expressing antibodies with desired sequences or functional properties are then isolated. In a preferred embodiment of the present invention, the murine antibody or fragments thereof may further comprise light chain constant regions of murine κ, λ chains or variants thereof, or further comprise heavy chain constant regions of murine IgG1, IgG2, IgG3 or IgG4 or variants thereof.

The term "human antibody" includes antibodies having variable and constant regions of human germline immunoglobulin sequences. The human antibody of the invention may include amino acid residues not encoded by human germline immunoglobulin sequences (e.g., mutations introduced by random or site-specific mutagenesis in vitro or by somatic mutation in vivo). However, the term "human antibody" does not include an antibody in which CDR sequence derived from the germline of another mammalian species, such as mouse, has been grafted onto human framework sequences (i.e., "humanized antibody").

The term "humanized antibody", also known as CDR-grafted antibody, refers to an antibody produced by grafting a CDR sequence of a murine into the frameworks of a human antibody variable region. It can overcome the intense immune response induced by a chimeric antibody carrying a large amount of mouse protein components. To avoid a decrease in activity caused by reducing the immunogenicity, the human antibody variable region can be subjected to minimal reverse mutation to maintain its activity.

The term "chimeric antibody" is an antibody formed by fusing a variable region of a murine antibody with a constant region of a human antibody, which can alleviate the immune response induced by a murine antibody. To construct the chimeric antibody, a hybridoma that secretes a murine-specific monoclonal antibody is first constructed and selected, then the variable region gene is cloned from the murine hybridoma cell. Subsequently, the constant region gene of the human antibody is cloned as needed. The murine variable region gene and the human constant region gene are ligated into a chimeric gene and then inserted into a human vector, and finally the chimeric antibody molecule is expressed in the eukaryotic or prokaryotic industrial system. The constant region of human antibody may be selected from the heavy chain constant region of human IgG1, IgG2, IgG3 or IgG4 or variants thereof, preferably comprising a heavy chain constant region of human IgG1 or IgG2, or IgG1 without ADCC (antibody-dependent cell-mediated cytotoxicity) toxicity after amino acid mutation.

The "antigen-binding fragment" described herein refers to a Fab fragment, a Fab' fragment, a F(ab')2 fragment with antigen-binding activity, or a Fv or sFv fragment bound to human GITR, which comprises one or more CDR regions of the antibodies described herein. An Fv fragment comprises a heavy chain variable region and a light chain variable region of an antibody (without constant regions), and has the smallest antibody fragment having all antigen binding sites. Generally, an Fv antibody also comprises polypeptide linkers between the VH and VL domains, and is capable of forming structures required for antigen binding. A variety of different linkers can be used to link two antibody variable regions into a polypeptide chain, called a single chain antibody or single chain Fv (sFv). The term "binding to GITR" of the present invention refers to being capable of interacting with human GITR. The term "antigen binding site" of the present invention refers to a discontinuous three-dimensional spatial site of an antigen, which is recognized by an antibody or antigen binding fragment of the present invention.

The term "epitope" refers to a site on the antigen that specifically binds to an immunoglobulin or antibody. An epitope can be formed by adjacent amino acids, or by non-adjacent amino acids juxtaposed by tertiary folding of proteins. Epitopes formed by adjacent amino acids are usually maintained upon exposure to denaturing solvents, while epitopes formed by tertiary folding are usually lost upon treatment with denaturing solvents. Epitopes usually comprise at least 3-15 amino acids in a unique spatial conformation. Methods for determining which epitopes are bound by a given antibody are well known in the art, comprising immunoblotting and immunoprecipitation assays etc. Methods for determining the spatial conformation of epitopes comprise techniques in the art and described in the present invention, such as X-ray crystallography and two-dimensional nuclear magnetic resonance.

The terms "specifically bind" or "selectively bind" used herein refer to the binding of an antibody to an epitope on a predetermined antigen. Usually, when recombinant human GITR is used as an analyte and its antibody is measured as a ligand by surface plasmon resonance (SPR) technology, the antibody binds to the predetermined antigen with an equilibrium dissociation constant (KD) of about 10⁻⁷M or even less, and its affinity to the predetermined antigen is at least twice its affinity to a non-specific antigen (for example, BSA, etc.) other than the predetermined antigen or a closely related antigen. The term "antibody(ies) that recognize(s) an antigen" can be used interchangeably herein with the term "antibody(ies) that specifically bind".

The term "competitive binding" refers to an antibody that recognizes and binds to the same epitope (also known as an antigenic determinant) or part of the same epitope on the extracellular domain of human GITR as the monoclonal antibody of the present invention does. An antibody that binds to the same epitope as the monoclonal antibody of the present invention refers to an antibody that recognizes the amino acid sequence of human GITR that is recognized by the monoclonal antibody of the present invention. The term "nucleic acid molecule" refers to DNA and RNA molecules. Nucleic acid molecules can be single-stranded or double-stranded, but preferably double-stranded DNA. When a nucleic acid molecule is placed in a functional relationship with another nucleic acid sequence, the nucleic acids are "effectively linked". For example, if promoters or enhancers affect the transcription of coding sequences, promoters or enhancers are effectively linked to the coding sequence.

The term "cross reactivity" refers to the ability of an antibody of the present invention to bind to GITR from different species. For example, an antibody that binds to human GITR of the present invention can also bind to GITR of another species. Cross-reactivity is measured in binding assays (such as SPR and ELISA) by detecting specific reactivity of antibodies with purified antigens or binding or functional interaction of antibodies with cells that physiologically express GITR. Methods for determining cross-reactivity comprise standard binding assays described herein, such as surface plasmon resonance (SPR) analysis or flow cytometry.

The terms "inhibit" and "block" can be used interchangeably and encompasses both partial and complete inhibition/blockade. The inhibition/blockade of CD70 preferably reduces or alters the normal level or type of activity that occurs when CD70 binds without inhibition or blockade. Inhibition and blockade also aim to reduce any measurable decrease in binding affinity of CD70 when exposed to anti-GITR antibodies as compared to CD70 that is not exposed to anti-GITR antibodies.

The term "inhibition of growth" (e.g. involving cells) is intended to include any measurable reduction in cell growth.

The terms "inducing immune response" and "enhancing immune response" can be used interchangeably and refer to the stimulation (i.e., passive or adaptive) of an immune response to specific antigens. The term "inducing", relating to inducing CDC or ADCC, refers to stimulating specific direct cellular killing mechanisms.

The "ADCC" described in the present invention, i.e. antibody-dependent cell-mediated cytotoxicity, refers to cells that express Fc receptors directly killing antibody-coated target cells by recognizing the Fc fragments of antibodies. The ADCC effect of an antibody can be reduced or eliminated by modifying the Fc fragment of IgG The modification refers to mutation(s) in the heavy chain constant region of of the antibody, e.g. mutation(s) selected from the group consisting of N297A, L234A, L235A; IgG2/4 chimera, F235E of IgG4, and L234A/E235A.

The fusion protein described in the present invention is a protein product obtained by co-expressing two genes through DNA recombination. The recombinant GITR extracellular domain-Fc fusion protein is a protein obtained through DNA recombination and co-expression of GITR extracellular domain and human antibody Fc fragment. The GITR extracellular domain refers to the part of GITR protein expressed on the outside portion of cell membrane.

Methods for producing and purifying antibody(ies) and antigen-binding fragments are well known and available in the art, such as Chapters 5-8 and 15 in Using Antibodies: A Laboratory Manual, Cold Spring Harbor. For example, mice can be immunized with human GITR or fragments thereof, and the obtained antibodies can be renatured, purified and subjected to amino acids sequencing using conventional methods. Antigen-binding fragments can also be prepared with conventional methods. The antibodies or antigen-binding fragments described herein are genetically engineered to introduce one or more human FR regions to the non-human CDR regions. Human FR germline sequences can be obtained from ImMunoGeneTics (IMGT) website http://imgt.cines.fr, or from The Immunoglobulin FactsBook (2001) ISBN:012441351. Specifically, the light chain FR germlines used for antibody or antigen-binding fragments of the present invention comprise Vk3-11 and Vk4-1. Specific heavy chain FR germlines used for antibody or antigen-binding fragments of the present invention comprise VH3-48 and VH1-2.

The engineered antibody or antigen-binding fragments herein can also be prepared and purified with conventional methods. For example, the cDNA sequences encoding heavy chain (SEQ ID NO: 9) and light chain (SEQ ID NO: 10) can be cloned and recombined into GS expression vectors. CHO cells can be stably transfected by the recombinant immunoglobulin expression vectors. As a more recommended technology, mammalian expression systems lead to glycosylation of antibodies, especially at the highly conserved N-terminus of the FC region. Stable clones are obtained by expressing antibodies that specifically bind to human antigens. Positive clones are expanded with serum-free medium in a bioreactor to produce antibodies. The culture solution, into which an antibody has been secreted, can be purified and collected via techniques known in the art. Antibodies can be filtered and concentrated via methods known in the art. Soluble mixtures and multimers can also be removed via methods known in the art, such as molecular sieve and ion exchange, and the resulting products need to be cryopreserved immediately, e.g. - 70°C, or lyophilized.

An antibody of the present invention refers to a monoclonal antibody. A monoclonal antibody (mAb) of the present invention refers to an antibody obtained from a single clonal cell strain, which is not limited to eukaryotic, prokaryotic or bacteriophage clonal cell strains. Monoclonal antibodies or antigen-binding fragments thereof can be obtained by hybridoma, recombination, phage display techniques, synthetic techniques (such as CDR-grafting), or recombination through other techniques in the art.

"Administration" and "treatment", when appling to animals, humans, experimental subjects, cells, tissues, organs or biological fluids, refer to contacting exogenous drugs, therapeutic agents, diagnostic agents or composition with animals, humans, subjects, cells, tissues, organs or biological fluids. "Administration" and "treatment" can refer to, for example, therapeutic, pharmacokinetics, diagnostic, research and experimental methods. Cell treatment includes contacting reagents with cells as well as contacting reagents with fluid, wherein the fluid are in contact with the cells. "Administration" and "treatment" also mean treating, for example, cells *in vitro* and *ex vivo* by reagents, diagnostics, binding compositions or another cell. "Treatment", when applied to human, veterinary or research subjects, refers to therapeutic treatment, prevention or preventive measures, research and diagnostic applications.

"Therapy" means administering to a patient an internal or external therapeutic agent, such as a composition comprising any of the binding compounds of the present invention, wherein the patient has one or more symptoms of the disease on which the therapeutic agent is known to have therapeutic effects. Usually, therapeutic agents are administered to the patients or population being treated in an amount that effectively alleviates the symptoms of one or more diseases, to the extent of either amelioration of the symptoms or inhibition of the development of the symptoms to any extent that is difficult to be measured clinically. The amount of therapeutic agents that effectively relieves symptoms of any specific disease (also known as the "therapeutically effective amount") can vary according to a number of factors, such as the patient's state of illness, age and weight, and the ability of the drug to elicit the desired efficacy in the patient. Whether the symptoms of the disease have been alleviated can be evaluated by any clinical test commonly used by doctors or other health care professionals to assess the severity or progression of the symptoms. Even if the embodiments of the present invention (e.g. therapeutic methods or preparations) may be ineffective in alleviating the symptoms of the target disease in each patient, it is determined by any statistics test known in the art, such as Student t test, Chi-square test, U test based on Mann and Whitney, Kruskal-Wallis test (H test), Jonckheere-Terpstra test and Wilcoxon test, that they are effective in alleviating the symptoms of the target disease in a statistically significant number of patients.

The term "essentially consist of" or variations thereof used in the entire specification and claims includes all such elements or element groups, and optionally inclueds other elements that are similar to or naturally different from said elements, wherein said other elements do not significantly alter the fundamental property or novel property of the designated dosing regimen, method or composition. As a non-limiting example, a binding compound consisting essentially of the amino acid sequence mentioned may also comprise one or more amino acids that do not significantly affect the properties of the binding compound.

The term "naturally occurring" applied to an object according to the present invention refers to the fact that the object can be found in nature. For example, living organisms (including viruses) that can be isolated from natural sources, and polypeptide or polynucleotide sequences which are not artificially modified in the laboratory are naturally occurring.

"Effective amount" comprises amounts sufficient to ameliorate or prevent the symptoms of a disease or a disorder. Effective amount also means amounts sufficient to allow or facilitate diagnosis. The effective amount for a particular patient or veterinary subject may vary depending on factors: e.g. the disease to be treated, the overall health condition of the patient, the route and dosage of the administration, and the severity of side effects. The effective amount may be the maximum dose or dosage regimen that avoids significant side effects or toxic effects.

"Exogenous" refers to substances that are produced outside of a living organism, cell or human body in the context of the invention. "Endogenous" refers to substances produced in organism, cell or human body in the context of the invention.

"Homology" refers to sequence similarity between two polynucleotide sequences or two polypeptides. When positions of two comparative sequences are occupied by the same base or amino acid monomer subunits, for example, if both positions of two DNA molecules are occupied by adenine, the molecules are homologous at this position. The percentage of homology between two sequences is a function of the number of matchings or homologous positions shared by two sequences divided by the number of comparative positions × 100%. For example, when the sequences are optimally aligned, if there are six matchings or homologous positions of 10 positions in the two sequences, then the two sequences are 60% homologous. Generally, comparisons are made when the maximum percentage of homology is obtained by aligning the two sequences.

The expressions "cell", "cell line" and "cell culture" used herein are interchangeable, and all such names include their progeny. Therefore, the words "transformants" and "transformed cells" comprise tested primary cells and cultures derived therefrom, regardless of the number of metastases. It should also be understood that it is impossible for all progeny to have exactly the same DNA content due to intentional or unintentional mutations, including the mutant progeny having the same function or biological activity as those screened in the original transformed cells, which is clear according to the context when referring to different names.

"Optional" or "optionally" means that an event or environment described subsequently may but does not necessarily occur, including situations where the event or environment occurs or does not occur. For example, "optionally comprising 1-3 antibody heavy chain variable regions" means that specific sequences of antibody heavy chain variable regions may but does not necessarily exist.

"Pharmaceutical composition" means a mixture comprising one or more of the compounds described herein, or physiological/medicinal salts thereof or a prodrug thereof, and other chemical components, as well as other components such as physiologically/medicinally acceptable carriers and excipients. The purpose of the pharmaceutical composition is to promote the administration of drugs to organisms and facilitate the absoption of active ingredients, thus exerting biological activity.

### Specific Modes of Carrying Out the Invention

The following embodiments further illustarte the present invention. However, these embodiments should not be limited to the scope of the present invention. Experimental methods without specified in the embodiments of the present invention are usually performed according to conventional conditions, such as Using Antibodies: A Laboratory Manual or Molecular Cloning: A Laboratory Manual, Cold Spring Harbor, or according to the conditions recommended by the manufacturers of raw materials or products. Reagents without specified source are commercially available conventional reagents.

### Example 1: Sequences and preparation of immune antigens and screened antigens

The sequences encoding human GITR with mFc tag (huGITR-mFc), GITR with hFc tag (huGITR-hFc) and GITRL with His × 6 tag (huGITRL-His6) were synthesized after optimization (the template sequences of all the GITR recombinant proteins above were designed in the present invention), and cloned into the pCDNA3.1 vector (purchased from Invirogen), respectively.
huGITR-mFc sequence 
MAQHGAMGAFRALCGLALLCALSLGQRPTGGPGCGPGRLLLGTGTDARCCRVH
TTRCCRDYPGEECCSEWDCMCVQPEFHCGDPCCTTCRHHPCPPGQGVQSQGKFSFGF
QCIDCASGTFSGGHEGHCKPWTDCTQFGFLTVFPGNKTHNAVCVPGSPPAEPKLENLYF
QGPRGPTIKPCPPCKCPAPNLLGGPSVFIFPPKIKDVLMISLSPIVTCVVVDVSEDDPDVQ
ISWFVNNVEVHTAQTQTHREDYNSTLRVVSALPIQHQDWMSGKEFKCKVNNKDLPAP
IERTISKPKGSVRAPQVYVLPPPEEEMTKKQVTLTCMVTDFMPEDIYVEWTNNGKTEL
NYKNTEPVLDSDGSYFMYSKLRVEKKNWVERNSYSCSVVHEGLHNHHTTKSFSRTPG
K
SEQ ID NO: 53
huGITR-hFc sequence 
MAQHGAMGAFRALCGLALLCALSLGQRPTGGPGCGPGRLLLGTGTDARCCRVH
TTRCCRDYPGEECCSEWDCMCVQPEFHCGDPCCTTCRHHPCPPGQGVQSQGKFSFGF
QCIDCASGTFSGGHEGHCKPWTDCTQFGFLTVFPGNKTHNAVCVPGSPPAEPKLENLYF
QGTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTP
 
PVLDSDGSFFLYSKLTVDKSRWQQGNVFSCSVMHEALHNHYTQKSLSLSPGK
SEQ ID NO: 54
huGITRL-His6 sequence

MGVLLTQRTLLSLVLALLFPSMASMQLETAKEPCMAKFGPLPSKWQMASSEPPCV
NKVSDWKLEILQNGLYLIYGQVAPNANYNDVAPFEVRLYKNKDMIQTLTNKSKIQNVG
GTYELHVGDTIDLIFNSEHQVLKNNTYWGIILLANPQFISHHHHHH
SEQ ID NO: 55

After transfecting 293F cells with the constructed recombinant plasmid for 7 days, the supernatant sample expressed by 293 cells was centrifuged at high speed prior to being filtered with a 0.45 µm membrane to remove impurities. A Protein A column was equilibrated with PBS solution and washed with 2-5 times the column volume prior to loading the supernantant sample onto the column. The column was washed with PBS solution until the A280 value decreased to the baseline. The target protein was eluted with 0.1M Citric (pH 3.2) solution, and the eluate peaks were neutralized with 1M Tris-HCl (pH 9.0). The neutralized eluate was replaced with PBS solution, and the harvested protein was identified by electrophoresis and then aliquoted for later use, thus obtaining the huGITR with mFc and hFc tags.

After transfecting 293F cells with the constructed recombinant plasmid for 7 days, the supernatant sample expressed by the 293 cells was centrifuged at high speed prior to being filtered with a 0.45 µm membrane to remove impurities. Imidazole was added to the supernatant to reach a final concentration of 5mM. A Nickel affinity column was equilibrated with 2-5 times the column volume of PBS solution containing 5mM of imidazole prior to loading the supernatant sample onto the column. The column was washed with PBS solution containing 5mM of imidazole until the A280 value decreased to the baseline. The chromatography column was then washed with PBS+10mM of imidazole to remove undesired non-specific binding proteins, and the flow-through was pooled. PBS solution containing 500mM of imidazole was then used to elute target proteins, and the eluates were pooled prior to being replaced with PBS solution, and the harvested protein was identified by electrophoresis prior to being aliquoted for later use, thus obtaining the huGITRL with His-tag.

### Example 2: Production of anti-human GITR monoclonal antibodies

Anti-human GITR monoclonal antibodies were produced by immunizing mice. 6 week old female BALB/c white mice were used (Guangdong Medical Laboratory Animal Center, **a**nimal production license number: 44007200025291), and they were fed in SPF facilities. After purchase, each group of 5 mice was fed for one week in the laboratory environment where the light/dark cycle was adjusted for 12/12 hours at a temperature of 20-25°C and a humidity of 40-60%. The immune antigen was human GITR recombinant protein (GITR-mFc) with mFc label. Groups were emulsified with Freund's adjuvant (sigma Lot Num:F5881/F5506). Complete Freund's adjuvant (CFA) was used for the prime immunization, and incomplete Freund's adjuvant (IFA) was used in the boost immunizations. The volume ratio of antigen to adjuvant was 1:1, the dose for prime immunization was 250 µl/50 µg/mouse, and the dose for boost immunization was 250 µl/50 µg/mouse as well. Emulsified antigen was inoculated on days 0, 14, 28 and 42. On day 0, 50 µg of emulsified antigen was injected subcutaneously (sc) at multiple sites into each mouse. On days 14, 28 and 42, antigen was injected dorsally or intraperitoneally depending on the conditions of the lumps on the back and the abdominal swelling. Immunization was boosted three days before splenocyte fusion.

Blood tests were performed on days 24, 38 and 52. The mouse serum was detected by the ELISA method described in Example 1 to determine the titers of antibodies in the mouse serum. Mice with high antibody titers in the serum were selected for spleen cell fusion, and spleen lymphocytes were fused with Sp2/0 myeloma cells (ATCC® CRL-1581™) by optimized PEG-mediated fusion procedure to obtain hybridoma cells. The ELISA method described in Example 3, binding assay for blocking GITRL described in Example 4 and cross-binding assay of mouse GITR and Cynomolgus GITR (cyno-GITR) described in Example 5 were used, and the results are shown in Table 1.

**Table 1: In vitro activity of mouse anti-GITR antibodies derived from different germlines**

| **Antibody Candidates** | **ELISA binding EC50 (nM)** | **GITRL blocking IC50 (nM)** | **ELISA binding EC50 (nM)** | |
|---|---|---|---|---|
| | | | cyno-GITR | mouse-GITR |
| 8A11 | 0.077 | 7.573 | 5.207 | No cross-binding |
| 18F10 | 0.103 | 6.037 | 0.744 | No cross-binding |
| 6A8 | 0.045 | 3.241 | N/A | N/A |
| 7D9 | 0.055 | 3.890 | N/A | N/A |
| 20H11 | 0.064 | 5.799 | N/A | N/A |
| 2B7 | 0.080 | 7.823 | N/A | N/A |
| 8H10 | 0.086 | 7.750 | N/A | N/A |
| 11C10 | 0.056 | 4.539 | N/A | N/A |
| 11F3 | 0.052 | 7.336 | N/A | N/A |
| 16G9 | 0.075 | 10.29 | N/A | N/A |
| BMAB reference | 0.074 | 1.386 | 0.269 | No cross-binding |

Monoclonal hybridoma cell lines with good *in vitro* activity were selected, and hybridoma cells in logarithmic growth phase were collected. RNA was extracted using RNAprep pure Cell / Bacteria Kit (Tiangen, catalog number: DP430) according to the user manual and reverse transcribed (PrimeScript Reverse Transcriptase, Takara, cat# 2680A). The reverse transcribed cDNA was amplified using mouse Ig-Primer Set (Novagen, TB326 Rev.B 0503) and PCR-amplified before subjected to commercial sequencing. After eliminating redundant or highly similar sequences with the same CDR, the mouse monoclonal antibody sequences 8A11, 18F10, 7D9 and 16G9 were obtained.

The heavy chain and light chain variable regions sequences of mouse monoclonal antibody 8A11 are as follows:
8A11 HCVR 
EVKLVESGGGLVKPGGSLKLSCAASGFTFSSYAMSWVRQTPEKRLEWVAYISSGG
STYYPDSVKGRFTISRENARNILYLQMSSLRSEDTAMYYCAREGYGTYGDYWGQGTT
LTVSS SEQ ID NO: 1
8A11 LCVR

DIVMSQSPSSLAVSVGEKVTMSCKSSQSLLYSTNQKNYLAWYQQKPGQSPKLLIY
WASTRESGVPDRFTGSGSGTDFTLTISSVKAEDLAVYYCQQYYSYLRTFGGGTKLEIK
SEQID NO: 2

The CDR sequences of 8A11 are as follows:

| Name | Sequence | NO. |
|---|---|---|
| HCDR1 | GFTFSSYAMS | SEQ ID NO: 3 |
| HCDR2 | YISSGGSTYYPDSVKG | SEQ ID NO: 4 |
| HCDR3 | EGYGTYGDY | SEQ ID NO: 5 |
| LCDR1 | KSSQSLLYSTNQKNYLA | SEQ ID NO: 6 |
| LCDR2 | WASTRES | SEQ ID NO: 7 |
| LCDR3 | QQYYSYLRT | SEQ ID NO: 8 |

The heavy chain and light chain variable regions sequences of mouse monoclonal antibody 18F10 are as follows:
18F10 HCVR 
QVQLQQSGAELVRPGVSVKISCKCSGYTFTGYDLHWVKQSHAKSLDWIGVISTY
YGDANYNQKFKGKATMTVDKSSSTAYMELARLTSEDSAIYYCTRLAESYFFDYWGQG
TTLTVSS SEQ ID NO: 9
18F10 LCVR

QILLTQSPAIMSASPGEKVTMTCRASSSVSYIHWYQQKPGSSPKPWIYDTSDLASG
FPARFSGTGSGTSYSLIISSMEAEDAATYYCHQRSSYPFTFGSGTKLEIK
SEQ ID NO: 10

The CDR sequences of 18F10 are as follows:

| Name | Sequence | NO. |
|---|---|---|
| HCDR1 | GYTFTGYDLH | SEQ ID NO: 11 |
| HCDR2 | VISTYYGDANYNQKFKG | SEQ ID NO: 12 |
| HCDR3 | LAESYFFDY | SEQ ID NO: 13 |
| LCDR1 | RASSSVSYIH | SEQ ID NO: 14 |
| LCDR2 | DTSDLAS | SEQ ID NO: 15 |
| LCDR3 | HQRSSYPFT | SEQ ID NO: 16 |

The heavy chain and light chain variable regions sequences of mouse monoclonal antibody 7D9 are as follows:
7D9 HCVR 
DVQLQESGPGLVKPSQSLSLTCTVTGYSITSDYAWNWIRQFPGNKLEWLGYISYSG
STYYNPSLKSRISLTRDTSKNQFFLQLNSVTSEDTATYYCARWDYRYDGRGFDYWGQG
TTLTVSS SEQ ID NO: 37
7D9 LCVR

DIQMTQSPSSLSASLGERVSLTCRASQDIGSGLNWLQQEPDGTIKRLIYATSSLDSG
VPKRFSGSRSGSDYSLTISSLESEDFVDYNCLQYTSTPWTFGGGTKLEIK

SEQ ID NO: 38

The CDR sequences of 7D9 are as follows:

| Name | Sequence | NO. |
|---|---|---|
| HCDR1 | GYSITSDYAW | SEQ ID NO: 39 |
| HCDR2 | YISYSGSTYYNPSLKS | SEQ ID NO: 40 |
| HCDR3 | WDYRYDGRGFDY | SEQ ID NO: 41 |
| LCDR1 | RASQDIGSGLN | SEQ ID NO: 42 |
| LCDR2 | ATSSLDS | SEQ ID NO: 43 |
| LCDR3 | LQYTSTPWT | SEQ ID NO: 44 |

The heavy chain and light chain variable regions sequences of mouse monoclonal antibody 16G9 are as follows:
16G9 HCVR 
QVQLQQSGAELAKPGASVKMSCKASGYTFTTYWIHWIKQRPGQGLEWIGYINPS
TGYTDYNQNFKDKATLTADQYSSTAYMQLSSLTFEDSAVYYCANYYGSTPYYWGRGT
TLTVSS SEQ ID NO: 45
16G9 LCVR

QIVLTQSPAIMSASPGEKVTMTCSASSSVTYVHWYQQKSGASPERWIFDASKLAS
GVPARFSGSGSGTSYSLTISSMETEDAATYYCQQWSSNPLTFGAGTKLELK
SEQ ID NO: 46

The CDR sequences of 16G9 are as follows:

| Name | Sequence | NO. |
|---|---|---|
| HCDR1 | GYTFTTYWIH | SEQ ID NO: 47 |
| HCDR2 | YINPSTGYTDYNQNFKD | SEQ ID NO: 48 |
| HCDR3 | YYGSTPYY | SEQ ID NO: 49 |
| LCDR1 | SASSSVTYVH | SEQ ID NO: 50 |
| LCDR2 | DASKLAS | SEQ ID NO: 51 |
| LCDR3 | QQWSSNPLT | SEQ ID NO: 52 |

### Example 3: ELISA binding assay

Anti-GITR antibodies block GITR binding to GITR-related receptors on the cell membrane by binding to GITR, thus blocking the downstream signaling pathways of GITR. ELISA was used to detect the binding specificities of anti-GITR antibodies. Microplates were coated with huGITR-mFc, and the signal intensity upon antibody addition was used to determine the binding activities of the antibodies and GITR.

The huGITR-mFc was diluted to 1 µg/ml with CBS buffer pH 9.5, added to the 96-well microplate at 50 µl/well, and incubated overnight at 4°C. The supernatant was discarded prior to 300 µl/well of 1% BSA blocking buffer diluted with PBS to the wells, and the plate was incubated for 2.5h at 37°C in an incubator for blocking. After blocking, the blocking buffer was discarded, and the plate was washed three times with PBST buffer (pH 7.4 PBS containing 0.05% tweeen-20). 50 µl/well of the antibodies to be tested were added, with the antibodies being diluted to different concentrations with sample diluent, and the plate was incubated at 37°C in an incubator for 30 miniutes. The plate was then washed with PBST three times, and 50 µL/well of Peroxidase AffiniPure Goat Anti-Human secondary antibody (Jackson immune research, 115-035-088) diluted with sample diluent was added, followed by incubating at 37°C for 30 minutes. After washing the plate with PBST three times, 50 µl/well of TMB chromogenic substrate was added and the plate was incubated at room temperature for 5 minutes prior to adding 50 µl/well of 1M H₂SO₄ to terminate the reaction. The absorption value at 450nm was read by a MD plus 384 microplate reader. The data were analyzed with SoftMax Pro6.2.1 to obtain binding EC50 values of the anti-GITR antibodies to GITR, as shown in Table 1.

### Example 4: anti-GITR antibodies block the binding of GITR and GITRL

GITR can bind to co-receptors of cell surface signaling pathways to inhibit activity of the pathways. In this experiment, blocking of the binding of human GITR to human GITRL by the screened anti-human GITR antibodies was tested using an *in vitro* blocking assay. Specifically, huGITR-hFc was coated to a 96-well microplate, followed by adding anti-GITR antibodies for pre-incubation, and GITRL-his protein was then added and co-incubated for 30 minutes. After washing the plate, the binding signals of GITRL and GITR were detected, and the EC50 values of anti-GITR antibodies blocking the active sites of GITR were analyzed with SoftMax Pro6.2. 1.

huGITR-mFc was diluted to 2 µg/ml with CBS buffer pH 9.5, added to the 96-well microplate at a volume of 50ul/well, and incubated overnight at 4°C. The supernatant was discarded prior to adding 300 µl/well of 1% BSA blocking buffer diluted with PBS, and the plate was incubated for blocking for 2.5h at 37°C in an incubator. After blocking, the blocking buffer was discarded, and the plate was washed three times with PBST buffer (pH 7.4 PBS containing 0.05% tween-20) prior to adding antibodies for pre-incubation, wherein the antibodies were diluted into gradient concentrations with sample diluent. 0.15µg/ml of GITRL-His protein in a volume of 50 µl/well was added, mixed and incubated at 37°C in an incubator for 30 miniutes. The reaction buffer was then discarded, the plate was washed with PBST three times, and 50 µL/well of Peroxidase AffiniPure Goat Anti-Human secondary antibody diluted with sample diluent at a ratio of 1:4000 was added, then the place was incubated at 37°C for 30 minutes. After washing the plate with PBST three times, 50 µl/well of TMB chromogenic substrate was added and the plate was incubated at room temperature for 5 minutes prior to adding 50 µl of 2M H₂SO₄ to terminate the reaction. The absorption value at 450nm was read by a MD plus 384 microplate reader. The EC50 values of GITR antibodies binding to GITR were obtained by analyzing the data with SoftMax Pro6.2.1, as shown in Table 1.

### Example 5: Cross-binding assay of anti-GITR antibodies and GITR derived from different species

To detect the *in vitro* binding ability of the screened anti-GITR antibodies to GITR from different species, mouse GITR and cynomolgus GITR were used for *in vitro* binding detection.

The GITR proteins from different species (mouse/cyno GITR) were diluted to 1 µg/ml with PBS buffer pH 9.5, and 50 µl/well was added to a 96-well microplate, which was incubated overnight (16-18 hours) at 4°C. The buffer in the plate was discarded prior to adding 300 µl/well of 1% BSA blocking buffer diluted with PBS, and the plate was incubated for blocking for 2.5h at 37°C in an incubator. After blocking, the blocking buffer was discarded, and the plate was washed three times with PBST buffer (pH 7.4 PBS containing 0.05% tweeen-20). 50 µl/well antibodies to be tested were added to the plate, wherein the antibodies were diluted to different concentrations with sample diluent (pH7.4 PBS), and the plate was incubated at 37°C in an incubator for 30 miniutes. After incubation, the reaction buffer in the microplate was discarded prior to washing the plate with PBST three times, and 50 µL/well of Peroxidase AffiniPure Goat Anti-Human secondary antibody (JacksonImmunoResearch, 109-035-088) which was diluted with sample diluent was added, and the plate was incubated at 37°C for 1 hour. After washing the plate with PBST six times, 50 µl/well of TMB chromogenic substrate (NEOGEN, 308177) was added, and the plate was incubated at room temperature for 5 minutes prior to adding 50 µl of 2M H₂SO₄ to terminate the reaction. The absorption value at 450nm was read by a MD plus 384 microplate reader. The EC50 values of GITR antibodies binding to GITR were calculated, as shown in Table 1.

### Example 6: In vitro binding affinity and dynamics assay

The affinities of humanized anti-GITR antibodies to be tested and human GITR were measured by Biacore, GE instruments.

A Protein A biosensor chip (Cat. 29-1275-56, GE) for a Biacore Instrument (Biacore T200, GE) was used to affinity-capture a certain amount of antibodies to be tested, and then a series of concentration gradients of GITR antigen (human h-GITR-his antigen was selected from Sino Biological Company (Cat.#13643-H08H)) were flowed through the surface of the chip. Real-time reaction signals were detected by a Biacore Instrument (Biacore T200, GE), thus obtaining the binding and dissociation curves. The biochip was rinsed and regenerated with 10 mM glycine pH 1.5 after each dissociation cycle, and the buffer was PBST, pH7.4. The experimental data were fitted with (1:1) Langmuir model using Biacore T200 Evaluation 2.0, GE software, and the affinity values were obtained, as shown in Table 2.

### Example 7: Cell activity assay of anti-GITR antibodies

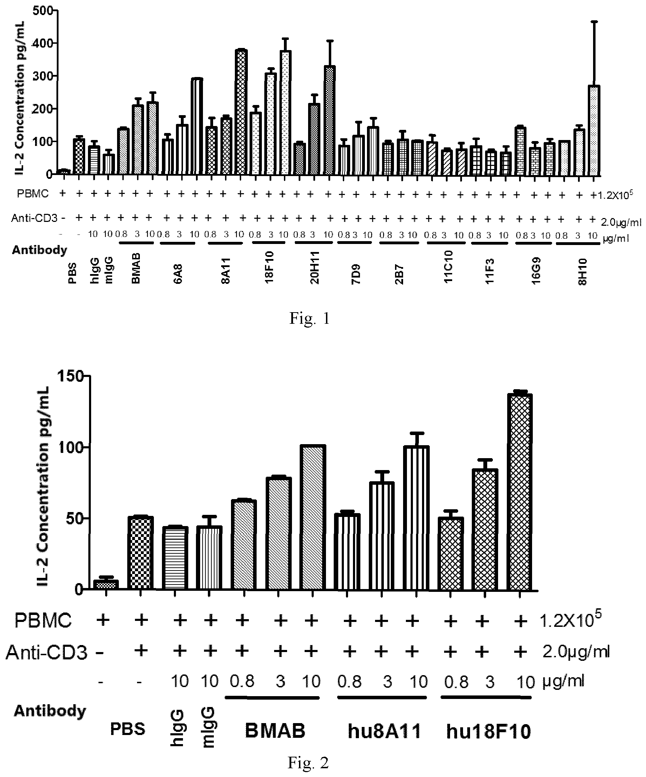

In this experiment, the activities of GITR antibodies *in vitro* were evaluated by detecting cell activity depending on the stimulated IL-2 secretion level. Anti-CD3 (2.0 µg/mL) was prepared and coated on microplates with 50 µL/well, and the plates were incubated overnight at 4°C. The next day, the plates were washed once with 300 µl/well of pre-cooled PBS, and the anti-GITR antibodies were diluted to experimentally designed concentrations with PBS. The diluted antibodies were coated on 96-well microplates at 50 µl/well (50 µl of PBS was added to the well of anti-CD3 control), and the plates were incubated at 37°C, 5% CO2 for 3h (only 300 µl of PBS was added to the periphery of the 96-well plate). The plates were washed once with 300 µ/welll of pre-cooled PBS (the well of anti-CD28 did not need to be washed). PBMCs were freshly isolated or resuscitated in 10% RPMI 1640 culture medium (resuscitated cells need to be cultured at 37°C for 2-3h), and 200 µl of the cell suspension with a density of 6 × 10⁵/ml and a culture medium of 10% RPMI 1640 medium were added to 96-well cell culture plates (2.0 µg/ml anti-CD28 antibody was added to wells costimulated by anti-CD28 antibody: wells coated with anti-CD3 but not coated with anti-GITR antibody). The cell culture plates were incubated in the incubator for 48h (37°C, 5% CO₂). 110µl/well of supernatant was collected, and the content of IL-2 was measured using an IL-2 ELISA kit. The experimental results of the *in vitro* cell activity of various mouse antibodies are shown in Figure 1, wherein the functional activity of 8A11, 18F10 and 20H11 is stronger than that of the reference antibody BMAB (Merck's MK-4166 antibody). (18F10 and 20H11 share exactly the same CDR sequences, so they are categorized as the same antibody).

### Example 8: Humanization of mouse antibodies

In this example, two strains with the strongest functional activity (8A11 and 18F10) among the obtained mouse antibodies were humanized. A human germline template with high homolgy was obtained by comparing the heavy and light chain variable region sequences with the antibody germline database on the basis of the typical structure of mouse antibody VH/VLCDR. The light chain framework regions of the human germline are derived from the human kappa light chain gene. For the antibody of the present invention, the light chain framework region is preferably the light chain template IGKV4-1^{∗}01 (8A11) and IGKV3-11^{∗}01 (18F10). The heavy chain framework region of the human germline is derived from the human heavy chain. For the antibody of the present invention, the heavy chain framework region is preferably human germline heavy chain template IGHV3-48^{∗}03 (8A11) and IGHV1-2^{∗}02 (18F10).

Human germline heavy chain template IGHV3-48*03 (SEQ ID NO: 21) :
EVQLVESGGGLVQPGGSLRLSCAASGFTFSSYEMNWVRQAPGKGLEWVSYISSSGSTI
YYADSVKGRFTISRDNAKNSLYLQMNSLRAEDTAVYYCAR

Human germline light chain template IGKV4-1*01 (SEQ ID NO: 22):
DIVMTQSPDSLAVSLGERATINCKSSQSVLYSSNNKNYLAWYQQKPGQPPKLLIYW
ASTRESGVPDRFSGSGSGTDFTLTISSLQAEDVAVYYCQQYYSTP

Human germline heavy chain template IGHV1-2*02 (SEQ ID NO: 23) :
QVQLVQSGAEVKKPGASVKVSCKASGYTFTGYYMHWVRQAPGQGLEWMGWIN
PNSGGTNYAQKFQGRVTMTRDTSISTAYMELSRLRSDDTAVYYCAR

Human germline light chain template IGKV3-11*01 (SEQ ID NO: 24):
EIVLTQSPATLSLSPGERATLSCRASQSVSSYLAWYQQKPGQAPRLLIYDASNRATG
IPARFSGSGSGTDFTLTISSLEPEDFAVYYCQQRSNWP

The CDR regions of mouse antibodies were grafted to the selected humanized template, thus replacing the humanized variable regions and recombining them with the constant regions of IgG. Then, based on the three-dimensional structure of mouse antibodies, the embedded residues, the residues with direct interaction with CDR regions and the residues having important influence on VL and VH conformations were reverse mutated, and the amino acid residues in the CDR regions were adjusted and optimized, wherein amino acid K in HCDR2: VISTYGDANYNQKFKG (SEQ ID NO: 12) was optimized to Q, and amino acid I in LCDR1: RASSSVSYIH (SEQID NO: 14) was optimized to L in mouse antibody 18F10, thus obtaing a series of humanized molecules. The heavy chain variable regions thereof are shown in SEQ ID NOs: 25-30; the light chain variable regions thereof are shown in SEQ ID NOs: 31-36, and the optimized CDR sequences are shown in SEQ ID NOs: 56-57.

The optimized CDR sequences of 18F10:

| Name | Sequence | NO. |
|---|---|---|
| HCDR2 | VISTYYGDANYNQKFQG | SEQ ID NO: 56 |
| LCDR1 | RASSSVSYLH | SEQ ID NO: 57 |

hu8A11-VH-a（SEQ ID NO: 25）:
EVQLVESGGGLVQPGGSLRLSCAASGFTFSSYAMSWVRQTPGKRLEWVAYISSGG
STYYPDSVKGRFTISRENAKNILYLQMNSLRAEDTAVYYCAREGYGTYGDYWGQGTT
LTVSS

hu8A11-VH-b（SEQ ID NO: 26）:
EVQLVESGGGLVQPGGSLRLSCAASGFTFSSYAMSWVRQAPGKGLEWVAYISSGG
STYYPDSVKGRFTISRDNAKNSLYLQMNSLRAEDTAVYYCAREGYGTYGDYWGQGTT
LTVSS

hu8A11-VH-c（SEQ ID NO: 27）:
EVQLVESGGGLVQPGGSLRLSCAASGFTFSSYAMSWVRQAPGKGLEWVSYISSGG
STYYADSVKGRFTISRDNAKNSLYLQMNSLRAEDTAVYYCAREGYGTYGDYWGQGTT
LTVSS

hu8A11-VL-a（SEQ ID NO: 28）:
DIVMTQSPSSLAVSVGERVTMSCKSSQSLLYSTNQKNYLAWYQQKPGQSPKLLIY
WASTRESGVPDRFSGSGSGTDFTLTISSVQAEDLAVYYCQQYYSYLRTFGGGTKLEIK

hu8A11-VL-b（SEQ ID NO: 29）:
DIVMTQSPSSLAVSVGERVTISCKSSQSLLYSTNQKNYLAWYQQKPGQSPKLLIYW

ASTRESGVPDRFSGSGSGTDFTLTISSLQAEDVAVYYCQQYYSYLRTFGGGTKLEIK

hu8A11-VL-c（SEQ ID NO: 30）:
DIVMTQSPSSLAVSVGERVTISCKSSQSLLYSTNQKNYLAWYQQKPGQPPKLLIYW
ASTRESGVPDRFSGSGSGTDFTLTISSLQAEDVAVYYCQQYYSYLRTFGGGTKLEIK

hu18F10-VH-a（SEQ ID NO: 31）:
QVQLVQSGAEVVKPGASVKISCKASGYTFTGYDLHWVKQAHGQGLDWIGVISTY
YGDATYNQKFQGRATMTVDTSSSTAYMELSRLTSEDTAVYYCTRLAESYFFDYWGQG
TTLTVSS

hu18F10-VH-b（SEQ ID NO: 32）:
QVQLVQSGAEVVKPGASVKVSCKASGYTFTGYDLHWVRQAPGQGLEWIGVISTY
YGDANYNQKFQGRVTMTVDTSSSTAYMELSRLRSEDTAVYYCTRLAESYFFDYWGQG
TTLTVSS

hu18F10-VH-c（SEQ ID NO: 33）:
QVQLVQSGAEVVKPGASVKVSCKASGYTFTGYDLHWVRQAPGQGLEWIGVISTY
YGDANYNQKFQGRVTMTVDTSISTAYMELSRLRSEDTAVYYCTRLAESYFFDYWGQG
TTLTVSS

hu18F10-VL-a（SEQ ID NO: 34）:
QIVLTQSPATLSASPGERVTMTCRASSSVSYIHWYQQKPGQAPKPWIYDTSNLASG
FPARFSGDGSGTDYSLIISSMEAEDAATYYCHQRSSYPFTFGSGTKLEIK

hu18F10-VL-b（SEQ ID NO: 35）:
EIVLTQSPATLSASPGERVTLTCRASSSVSYLHWYQQKPGQAPKPWIYDTSDLASG
FPARFSGDGSGTDYTLTISSLEAEDAAVYYCHQRSSYPFTFGSGTKLEIK

hu18F10-VL-c（SEQ ID NO: 36）:
EIVLTQSPATLSASPGERVTLTCRASSSVSYLHWYQQKPGQAPKPLIYDTSDLASGI
PARFSGDGSGTDYTLTISSLEAEDAAVYYCHQRSSYPFTFGSGTKLEIK

The final humanized hu8A11 (using VH-b heavy chain and VL-b light chain) and hu18F10 antibody molecule (using VH-b heavy chain and VL-b light chain) were comprehensively evaluated and selected via tests of small-scale expression and comparison of the number of reverse mutations of combination of the above light and heavy chains, and the sequences of their full-length light and heavy chains are shown in SEQ ID NOs: 17-20.
hu8A11 HC 
EVQLVESGGGLVQPGGSLRLSCAASGFTFSSYAMSWVRQAPGKGLEWVAYISSGG
STYYPDSVKGRFTISRDNAKNSLYLQMNSLRAEDTAVYYCAREGYGTYGDYWGQGTT
LTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGK
SEQ ID NO: 17
hu8A11 LC 
DIVMTQSPSSLAVSVGERVTISCKSSQSLLYSTNQKNYLAWYQQKPGQSPKLLIYW
ASTRESGVPDRFSGSGSGTDFTLTISSLQAEDVAVYYCQQYYSYLRTFGGGTKLEIKRTV
AAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID NO: 18
hu18F10 HC 
QVQLVQSGAEVVKPGASVKVSCKASGYTFTGYDLHWVRQAPGQGLEWIGVISTY
YGDANYNQKFQGRVTMTVDTSSSTAYMELSRLRSEDTAVYYCTRLAESYFFDYWGQG
TTLTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCPP
CPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPGK
SEQ ID NO: 19
hu18F10 LC

EIVLTQSPATLSASPGERVTLTCRASSSVSYLHWYQQKPGQAPKPWIYDTSDLASG
FPARFSGDGSGTDYTLTISSLEAEDAAVYYCHQRSSYPFTFGSGTKLEIKRTVAAPSVFIF
PPSDEQLKSGTASVVCLLNNFYPREAKVQWKVDNALQSGNSQESVTEQDSKDSTYSLS
STLTLSKADYEKHKVYACEVTHQGLSSPVTKSFNRGEC

SEQ ID NO: 20

### Example 9: Test data of humanized antibody activity

The constructed recombinant plasmids encompassing humanized heavy chain and light chain (molar ratio 1:1) was transfected into 293F cells. After 7 days, the supernatant sample expressed by 293 cells was centrifuged at high speed prior to being filtered through a 0.45 µm membrane to remove impurities. A Protein A column was equilibrated with PBS solution and washed with 2-5 times the column volume. The supernatant was loaded onto the column, which was washed with PBS solution until the A280 value decreased to baseline. The target protein was eluted by 0.1M Citric (pH 3.2) solution, and the pooled elution peaks were neutralized by 1M Tris-HCl (pH 9.0). The neutralized eluate was exchanged into PBS solution, and the obtained protein was identified by SEC-HPLC and was aliquoted for later use.

The final humanized versions of antibody molecules hu8A11 and hu18F10 were tested by *in vitro* binding affinity and kinetics experiments described in Example 6 and cell activity experiments described in Example 7, and compared with Merck's reference antibody BMAB. The results are shown in Table 2 and Fig. 2, respectively.

**Table 2: Affinity kinetics data of humanized antibody in vitro**

| Antibody | Antigen | Association Velocity kₐ(1/M*s) | Dissociation Velocity k_{d} (1/s) | Affinity K_{D} |
|---|---|---|---|---|
| hu8A11 | h-GITR-his | 1.68E+05 | 1.04E-04 | 0.62nM |
| hu18F10 | h-GITR-his | 6.13E+05 | 2.04E-04 | 0.33nM |
| BMAB reference | h-GITR-his | 2.72E+05 | 5.01E-04 | 185pM |

The above results show that the humanized anti-human GITR antibodies retain the binding activity before humanization, have equivalent or stronger cellular functional activity as compared to that of the BMAB reference, and have a strong binding affinity *in vitro.*

### Example 10: Inhibition of tumor cell growth by anti-GITR antibody hu8A11

PBMC was isolated from the peripheral blood of healthy individuals by density gradient centrifugation. Monocytes were sorted using a CD14+ microbeads kit, mixed with A375 melanoma cells (Cell Bank of Shanghai Institutes for Biological Sciences, cultured in DMEM medium containing 10% fetal bovine serum), and inoculated subcutaneously into NCG mice (purchased from Nanjing Biomedical Research Institute of Nanjing University and fed for 5 days to adapt to the environment). The experimental animals were all fed in an independent ventilated box with constant temperature and humidity. The temperature of the feeding room was 18.0-26.0°C, the humidity was 40-70%, and the ventilation frequency was 10-20 times per hour. The day and night shift was 12h/12h.

The experiment subjects were divided into a human IgG1 antibody control group, three constant dosages of hu8A11 groups (250 µg, 500 µg, 1000 µg) and a reference antibody BMAB group (1000 µg). Eight mice in each group were administered via subcutaneous injection. During the duration of the experiment, the length and width of the tumors were measured twice a week with a caliper, and the volume of the tumors were calculated according to the following formula: tumor volume (mm³) = 0.5 × ( tumor length × tumor width²). Relative tumor growth inhibiton rate TGI (%): TGI%=(1-HC)×100% T/C% is the relative tumor proliferation rate, i.e. at a certain time point, the percent ratio of the relative tumor volume or weight of the treatment group and the control group. T and C were tumor volume (TV) or tumor weight (TW) at a specific time point in the treatment group and IgG1 control group, respectively.

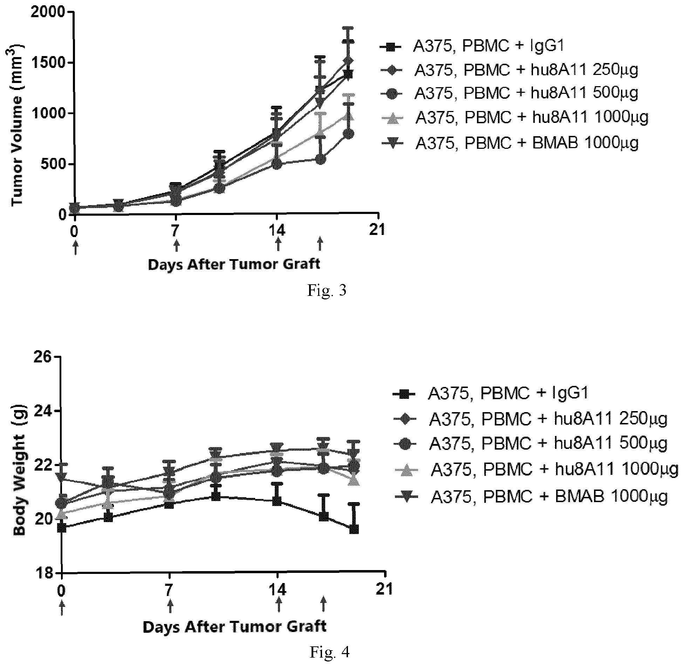

Results are shown in Table 3, Fig. 3 and Fig. 4. Humanized anti-GITR antibody hu8A11 showed a significant antitumor effect compared with IgG1 control, and its antitumor effect was significantly stronger than that of the same dose of reference antibody BMAB.

**Table 3: Tumor growth in NCG mice bearing A375 melanoma (mm³) co-transplanted with human PBMC**

| Days after tumor graft | IgG1 | | 250µg hu8A11 | | 500µg hu8A11 | | 1000µg hu8A11 | | 1000µg BMAB | |
|---|---|---|---|---|---|---|---|---|---|---|
| | MEAN | SE | MEAN | SE | MEAN | SE | MEAN | SE | MEAN | SE |
| Day 3 | 101.05 | 20.89 | 95.20 | 17.51 | 80.81 | 23.06 | 78.86 | 14.96 | 101.48 | 20.50 |
| Day 7 | 226.41 | 70.90 | 204.22 | 49.40 | 126.96 | 47.66 | 140.61 | 46.91 | 205.81 | 68.90 |
| Day 10 | 464.47 | 150.05 | 403.53 | 117.32 | 251.09 | 116.83 | 258.77 | 64.63 | 421.21 | 140.24 |
| Day 14 | 799.30 | 245.29 | 778.57 | 201.76 | 484.49 | 189.94 | 553.26 | 139.81 | 736.08 | 199.28 |
| Day 17 | 1211.07 | 329.55 | 1206.17 | 285.96 | 535.55 | 208.55 | 796.11 | 182.18 | 1082.74 | 255.62 |
| Day 19 | 1368.09 | 325.08 | 1497.18 | 326.09 | 777.65 | 298.48 | 968.91 | 194.34 | 1354.61 | 324.21 |

Although the specific embodiments of the present invention have been described above, it should be understood by those skilled in the art that these embodiments can be varied or modified in multiple ways without departing from the principles and essence of the present invention. Therefore, the scope of protection of the present invention is limited by the appended claims.

## Claims

1. An anti-GITR antibody or antigen-binding fragment thereof, comprising:
an antibody light chain variable region comprising at least one LCDR selected from the group consisting of SEQ ID NO: 6, SEQ ID NO: 7, SEQ ID NO: 8; SEQ ID NO: 57, SEQ ID NO. 14, SEQ ID NO: 15, SEQ ID NO: 16; SEQ ID NO: 42, SEQ ID NO: 43, SEQ ID NO: 44; SEQ ID NO: 50, SEQ ID NO: 51 and SEQ ID: 52; and/or
an antibody heavy chain variable region comprising at least one HCDR selected from the group consisting of SEQ ID NO: 3, SEQ ID NO:4, SEQ ID NO:5; SEQ ID NO: 11, SEQ ID NO:12, SEQ ID NO:56, SEQ ID NO:13; SEQ ID NO:39, SEQ ID NO:40, SEQ ID NO:41; SEQ ID NO:47, SEQ ID NO:48 and SEQ ID NO:49.

2. The anti-GITR antibody or antigen-binding fragment thereof according to claim 1, wherein the LCDR comprises one or more of LCDR1, LCDR2 and LCDR3, the amino acid sequence of the LCDR1 is shown in SEQ ID NO:6, SEQ ID NO:14, SEQ ID NO:57, SEQ ID NO: 42 or SEQ ID NO:50; the amino acid sequence of the LCDR2 is shown in SEQ ID NO:7, SEQ ID NO:15, SEQ ID NO: 43 or SEQ ID NO:51; the amino acid sequence of the LCDR3 is shown in SEQ ID NO:8, SEQ ID NO:16, SEQ ID NO: 44 or SEQ ID NO:52;
and/or, the HCDR comprises one or more of HCDR1, HCDR2 and HCDR3, the amino acid sequence of the HCDR1 is shown in SEQ ID NO:3, SEQ ID NO:11, SEQ ID NO: 39 or SEQ ID NO:47; the amino acid sequence of the HCDR2 is shown in SEQ ID NO: 4, SEQ ID NO:12, SEQ ID NO: 56, SEQ ID NO:40 or SEQ ID NO:48; the amino acid sequence of HCDR3 the is shown in SEQ ID NO:5, SEQ ID NO:13, SEQ ID NO: 41or SEQ ID NO:49.

3. The anti-GITR antibody or antigen-binding fragment thereof according to claim 2, wherein the antibody light chain variable region comprises the LCDR1, LCDR2 and LCDR3 as shown in SEQ ID NO: 6, SEQ ID NO:7 and SEQ ID NO:8, respectively; or the LCDR1, LCDR2 and LCDR3 as shown in SEQ ID NO: 57, SEQ ID NO:15 and SEQ ID NO:16, respectively; or the LCDR1, LCDR2 and LCDR3 as shown in SEQ ID NO:14, SEQ ID NO:15 and SEQ ID NO:16, respectively; or the LCDR1, LCDR2 and LCDR3 as shown in SEQ ID NO: 42, SEQ ID NO:43 and SEQ ID NO:44, respectively; or the LCDR1, LCDR2 and LCDR3 as shown in SEQ ID NO: 50, SEQ ID NO:51 and SEQ ID NO:52, respectively;
and/or the antibody heavy chain variable region comprises the HCDR1, HCDR2 and HCDR3 as shown in SEQ ID NO: 3, SEQ ID NO:4 and SEQ ID NO:5, respectively; or the HCDR1, HCDR2 and HCDR3 as shown in SEQ ID NO: 11, SEQ ID NO:56 and SEQ ID NO:13, respectively; or the HCDR1, HCDR2 and HCDR3 as shown in SEQ ID NO: 11, SEQ ID NO:12 and SEQ ID NO:13, respectively; or the HCDR1, HCDR2 and HCDR3 as shown in SEQ ID NO: 39, SEQ ID NO:40 and SEQ ID NO:41, respectively; or the HCDR1, HCDR2 and HCDR3 as shown in SEQ ID NO: 47, SEQ ID NO:48 and SEQ ID NO:49, respectively.

4. The anti-GITR antibody or antigen-binding fragment thereof according to claim 3, wherein the antibody light chain variable region comprises the LCDR1, LCDR2 and LCDR3 as shown in SEQ ID NO:6, SEQ ID NO:7 and SEQ ID NO:8, respectively; and the heavy chain variable region comprises the HCDR1, HCDR2 and HCDR3 as shown in SEQ ID NO:3, SEQ ID NO:4 and SEQ ID NO:5, respectively;
and/or the antibody light chain variable region comprises the LCDR1, LCDR2 and LCDR3 as shown in SEQ ID NO:57, SEQ ID NO:15 and SEQ ID NO:16, respectively; and the heavy chain variable region comprises the HCDR1, HCDR2 and HCDR3 as shown in SEQ ID NO:11, SEQ ID NO:56 and SEQ ID NO:13, respectively;
and/or the antibody light chain variable region comprises the LCDR1, LCDR2 and LCDR3 as shown in SEQ ID NO:14, SEQ ID NO:15 and SEQ ID NO:16, respectively; and the heavy chain variable region comprises the HCDR1, HCDR2 and HCDR3 as shown in SEQ ID NO:11, SEQ ID NO:12 and SEQ ID NO:13, respectively;
and/or the antibody light chain variable region comprises the LCDR1, LCDR2 and LCDR3 as shown in SEQ ID NO:42, SEQ ID NO:43 and SEQ ID NO:44, respectively; and the heavy chain variable region comprises the HCDR1, HCDR2 and HCDR3 as shown in SEQ ID NO:39, SEQ ID NO:40 and SEQ ID NO:41, respectively;
and/or the antibody light chain variable region comprises the LCDR1, LCDR2 and LCDR3 as shown in SEQ ID NO:50, SEQ ID NO:51 and SEQ ID NO:52, respectively; and the heavy chain variable region comprises the HCDR1, HCDR2 and HCDR3 as shown in SEQ ID NO:47, SEQ ID NO:48 and SEQ ID NO:49, respectively.

5. The anti-GITR antibody or antigen-binding fragment thereof according to any of claims 1-4, wherein the antibody or antigen-binding fragment thereof is a murine antibody, or a chimeric antibody.

6. The anti-GITR antibody or antigen-binding fragment thereof according to claim 5, wherein light chain variable region LCVR of the murine antibody or the chimeric antibody is SEQ ID NO:2, and heavy chain variable region HCVR is SEQ ID NO: 1;
or light chain variable region LCVR is SEQ ID NO:10 and heavy chain variable region HCVR is SEQ ID NO:9;
or light chain variable region LCVR is SEQ ID NO:38 and heavy chain variable region HCVR is SEQ ID NO:37;
or light chain variable region LCVR is SEQ ID NO:46 and heavy chain variable region HCVR is SEQ ID NO:45.

7. The anti-GITR antibody or antigen-binding fragment thereof according to any of claims 1-4, wherein the antibody or antigen-binding fragment thereof is a human antibody or a humanized antibody.

8. The anti-GITR antibody or antigen-binding fragment thereof according to claim 7, wherein the sequence of light chain FR region of the humanized antibody light chain variable region is derived from light chain IGkV4-1 sequence of the human germline as shown in SEQ ID NO: 22, or from light chain IGkV3-11 sequence of the human germline as shown in SEQ ID NO: 24.

9. The anti-GITR antibody or antigen-binding fragment thereof according to claim 7, wherein the antibody light chain sequence is shown in SEQ ID NO:18 or SEQ ID NO: 20 or variants thereof; the variant preferably has 0-10 amino acid mutation in the light chain, wherein the amino acid sequence of SEQ ID NO: 18 is preferably mutated at position 49, and the mutation of amino acid residue is preferably S49P; wherein the amino acid sequences of SEQ ID NO: 20 are preferably muatated at positions 46, 52 and 57, and the mutation of amino acid residues are preferably W46L, D52N and F57I.

10. The anti-GITR antibody or antigen-binding fragment thereof according to any of claims 7-9, wherein the humanized antibody heavy chain variable region comprises heavy chain FR region of human IgG1, IgG2, IgG3 or IgG4 or variants thereof, preferably comprising the heavy chain FR region of human IgG1, IgG2 or IgG4, more preferably comprising the heavy chain FR region of human IgG1 or IgG2.

11. The anti-GITR antibody or antigen-binding fragment thereof according to claim 10, wherein the sequence of the heavy chain FR region of the humanized antibody heavy chain variable region is derived from heavy chain IGHV3-48 sequence of the human germline as shown in SEQ ID NO:21, or from heavy chain IGHV1-2 sequence of the human germline as shown in SEQ ID NO:23.

12. The anti-GITR antibody or antigen-binding fragment thereof according to any of claims 7-11, wherein the humanized antibody heavy chain sequence is shown as SEQ ID NO: 17 or SEQ ID NO: 19 or its variant; the variant preferably has 0-10 amino acid alteration in the heavy chain; wherein the amino acid sequence of SEQ ID NO: 17 is preferably 49, and the mutation of amino acid residue is preferably A49S; wherein the amino acid sequences of SEQ ID NO: 19 are preferably mutated at positions 61 and 76, and mutation of amino acid residues are preferably N61A and S76I.

13. The anti-GITR antibody or antigen-binding fragment thereof according to any of claims 8-12, wherein the antibody is a humanized antibody hu18F10 or a humanized antibody hu8A11, wherein the variable regions of the humanized antibody are as follows:
humanized antibody hu18F10: the sequence of the heavy chain variable region is shown in SEQ ID NO:33; the sequence of the light chain variable region is shown in SEQ ID NO:36;
humanized antibody hu8A11: the sequence of the heavy chain variable region is shown in SEQ ID NO:26; the sequence of the light chain variable region is shown in SEQ ID NO:29.

14. The anti-GITR antibody or antigen-binding fragment thereof according to claim 13, wherein the humanized antibody hu18F10 comprises the sequence of heavy chain as shown in SEQ ID NO:19 and the sequence of light chain as shown in SEQ ID NO:20; wherein the humanized antibody h18A11 comprises the sequence of heavy chain as shown in SEQ ID NO:17 and the sequence of light chain as shown in SEQ ID NO:18.

15. An isolated monoclonal antibody or antigen-binding fragment thereof, which can compete with the anti-GITR antibody or antigen-binding fragment thereof according to any of claims 1-14 in binding GITR.

16. A multi-specific antibody, comprising the light chain variable region or the heavy chain variable region of the anti-GITR antibody or antigen-binding fragment thereof according to any of claims 1-14 or the isolated monoclonal antibody or antigen-binding fragment thereof according to claim 15.

17. A single chain antibody, comprising the light chain variable region or the heavy chain variable region of the anti-GITR antibody or antigen-binding fragment thereof according to any of claims 1-14 or the isolated monoclonal antibody or antigen-binding fragment thereof according to claim 15.

18. An antibody-drug conjugate, comprising the light chain variable region and/or the heavy chain variable region of the anti-GITR antibody or antigen-binding fragment thereof according to any of claims 1-14 or the isolated monoclonal antibody or antigen-binding fragment thereof according to claim 15.

19. A nucleic acid encoding the anti-GITR antibody or antigen-binding fragment thereof according to any of claims 1-14, the multi-specific antibody according to claim 16, the single chain antibody according to claim 17.

20. An expression vector, comprising the nucleic acid according to claim 19.

21. A host cell transformed with the expression vector according to claim 20.

22. The host cell according to claim 21, wherein the host cell is a bacteria, preferably *Eshcerichia coli*.; or the host cell is a yeast, preferably *Pichia pastoris*; or the host cell is a mammalian cell, preferably Chinese hamster ovarian (CHO) cell or human embryo kidney (HEK) 293 cell.

23. A method of preparing the anti-GITR antibody or antigen-binding fragment thereof according to any of claims 1-14, comprising expressing the anti-GITR antibody or antigen-binding fragment thereof in the host cell according to claim 22, and isolating the anti-GITR antibody or antigen-binding fragment thereof from the host cell.

24. A pharmaceutical composition, comprising the anti-GITR antibody or antigen-binding fragment thereof according to any of claims 1-14, the isolated monoclonal antibody or antigen-binding fragment thereof according to claim 15, the multi-specific antibody according to claim 16 or the single chain antibody according to claim 17, and medicinal excipients, diluents or carriers.

25. The anti-GITR antibody or antigen-binding fragment thereof according to any of claims 1-14, the isolated monoclonal antibody or antigen-binding fragment thereof according to claim 15, the multi-specific antibody according to claim 16, the single chain antibody according to claim 17 or the pharmaceutical composition according to claim 24 for use in the preparation of drugs for treating GITR or GITRL-mediated diseases or disorders; wherein the disease is preferably cancer; the cancer is most preferably melanoma, breast cancer, ovarian cancer, prostate cancer, pancreatic cancer, renal cancer, lung cancer, liver cancer, gastric cancer, colorectal cancer, bladder cancer, esophageal cancer, cervical cancer, multiple myeloma, leukemia, lymphoma, gallbladder cancer and glioblastoma.

26. A method for treating and preventing GITR or GITRL-mediated diseases or disorders, comprising administering to the patient in need thereof a therapeutically effective amount of the anti-GITR antibody or antigen-binding fragment thereof according to any of claims 1-14, the isolated monoclonal antibody or antigen-binding fragment thereof according to claim 15, the multi-specific antibody according to claim 16, the single chain antibody according to claim 17 or the pharmaceutical composition according to claim 24, wherein the disease is preferably cancer; the cancer is most preferably melanoma, breast cancer, ovarian cancer, prostate cancer, pancreatic cancer, renal cancer, lung cancer, liver cancer, gastric cancer, colorectal cancer, bladder cancer, esophageal cancer, cervical cancer, multiple myeloma, leukemia, lymphoma, gallbladder cancer and glioblastoma.


SEQUENCE LISTING

<110>  JIANGSU HENGRUI MEDICINE CO., LTD.
       SHANGHAI HENGRUI PHARMACEUTICAL CO., LTD

<120>  Anti-GITR Antibody, Antigen-binding Fragment Thereof, and
       Pharmaceutical Use Thereof

<130>  P19413703EP

<140>  PCT/CN2018/093572
<141>  2018-06-29

<150>  CN201710522742.4
<151>  2017-06-30

<160>  57

<170>  PatentIn version 3.5

<210>  1
<211>  117
<212>  PRT
<213>  Mus musculus

<400>  1

Glu Val Lys Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Ala Met Ser Trp Val Arg Gln Thr Pro Glu Lys Arg Leu Glu Trp Val
        35                  40                  45

Ala Tyr Ile Ser Ser Gly Gly Ser Thr Tyr Tyr Pro Asp Ser Val Lys
    50                  55                  60

Gly Arg Phe Thr Ile Ser Arg Glu Asn Ala Arg Asn Ile Leu Tyr Leu
65                  70                  75                  80

Gln Met Ser Ser Leu Arg Ser Glu Asp Thr Ala Met Tyr Tyr Cys Ala
                85                  90                  95

Arg Glu Gly Tyr Gly Thr Tyr Gly Asp Tyr Trp Gly Gln Gly Thr Thr
            100                 105                 110

Leu Thr Val Ser Ser
        115


<210>  2
<211>  113
<212>  PRT
<213>  Mus musculus

<400>  2

Asp Ile Val Met Ser Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Ser Tyr Leu Arg Thr Phe Gly Gly Gly Thr Lys Leu Glu Ile
            100                 105                 110

Lys

<210>  3
<211>  10
<212>  PRT
<213>  Mus musculus

<400>  3

Gly Phe Thr Phe Ser Ser Tyr Ala Met Ser
1               5                   10

<210>  4
<211>  16
<212>  PRT
<213>  Mus musculus

<400>  4

Tyr Ile Ser Ser Gly Gly Ser Thr Tyr Tyr Pro Asp Ser Val Lys Gly
1               5                   10                  15

<210>  5
<211>  9
<212>  PRT
<213>  Mus musculus

<400>  5

Glu Gly Tyr Gly Thr Tyr Gly Asp Tyr
1               5

<210>  6
<211>  17
<212>  PRT
<213>  Mus musculus

<400>  6

Lys Ser Ser Gln Ser Leu Leu Tyr Ser Thr Asn Gln Lys Asn Tyr Leu
1               5                   10                  15

Ala

<210>  7
<211>  7
<212>  PRT
<213>  Mus musculus

<400>  7

Trp Ala Ser Thr Arg Glu Ser
1               5

<210>  8
<211>  9
<212>  PRT
<213>  Mus musculus

<400>  8

Gln Gln Tyr Tyr Ser Tyr Leu Arg Thr
1               5

<210>  9
<211>  118
<212>  PRT
<213>  Mus musculus

<400>  9

Gln Val Gln Leu Gln Gln Ser Gly Ala Glu Leu Val Arg Pro Gly Val
1               5                   10                  15

Ser Val Lys Ile Ser Cys Lys Cys Ser Gly Tyr Thr Phe Thr Gly Tyr
            20                  25                  30

Asp Leu His Trp Val Lys Gln Ser His Ala Lys Ser Leu Asp Trp Ile
        35                  40                  45

Gly Val Ile Ser Thr Tyr Tyr Gly Asp Ala Asn Tyr Asn Gln Lys Phe
    50                  55                  60

Lys Gly Lys Ala Thr Met Thr Val Asp Lys Ser Ser Ser Thr Ala Tyr
65                  70                  75                  80

Met Glu Leu Ala Arg Leu Thr Ser Glu Asp Ser Ala Ile Tyr Tyr Cys
                85                  90                  95

Thr Arg Leu Ala Glu Ser Tyr Phe Phe Asp Tyr Trp Gly Gln Gly Thr
            100                 105                 110

Thr Leu Thr Val Ser Ser
        115

<210>  10
<211>  106
<212>  PRT
<213>  Mus musculus

<400>  10

Gln Ile Leu Leu Thr Gln Ser Pro Ala Ile Met Ser Ala Ser Pro Gly
1               5                   10                  15

Glu Lys Val Thr Met Thr Cys Arg Ala Ser Ser Ser Val Ser Tyr Ile
            20                  25                  30

His Trp Tyr Gln Gln Lys Pro Gly Ser Ser Pro Lys Pro Trp Ile Tyr
        35                  40                  45

Asp Thr Ser Asp Leu Ala Ser Gly Phe Pro Ala Arg Phe Ser Gly Thr
    50                  55                  60

Gly Ser Gly Thr Ser Tyr Ser Leu Ile Ile Ser Ser Met Glu Ala Glu
65                  70                  75                  80

Asp Ala Ala Thr Tyr Tyr Cys His Gln Arg Ser Ser Tyr Pro Phe Thr
                85                  90                  95

Phe Gly Ser Gly Thr Lys Leu Glu Ile Lys
            100                 105

<210>  11
<211>  10
<212>  PRT
<213>  Mus musculus

<400>  11

Gly Tyr Thr Phe Thr Gly Tyr Asp Leu His
1               5                   10

<210>  12
<211>  17
<212>  PRT
<213>  Mus musculus

<400>  12

Val Ile Ser Thr Tyr Tyr Gly Asp Ala Asn Tyr Asn Gln Lys Phe Lys
1               5                   10                  15

Gly

<210>  13
<211>  9
<212>  PRT
<213>  Mus musculus

<400>  13

Leu Ala Glu Ser Tyr Phe Phe Asp Tyr
1               5

<210>  14
<211>  10
<212>  PRT
<213>  Mus musculus

<400>  14

Arg Ala Ser Ser Ser Val Ser Tyr Ile His
1               5                   10

<210>  15
<211>  7
<212>  PRT
<213>  Mus musculus

<400>  15

Asp Thr Ser Asp Leu Ala Ser
1               5

<210>  16
<211>  9
<212>  PRT
<213>  Mus musculus

<400>  16

His Gln Arg Ser Ser Tyr Pro Phe Thr
1               5

<210>  17
<211>  447
<212>  PRT

<213>  Artificial sequence

<220>
<223>  Humanized antibody

<400>  17

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ala Tyr Ile Ser Ser Gly Gly Ser Thr Tyr Tyr Pro Asp Ser Val Lys
    50                  55                  60

Gly Arg Phe Thr Ile Ser Arg Asp Asn Ala Lys Asn Ser Leu Tyr Leu
65                  70                  75                  80

Gln Met Asn Ser Leu Arg Ala Glu Asp Thr Ala Val Tyr Tyr Cys Ala
                85                  90                  95

Arg Glu Gly Tyr Gly Thr Tyr Gly Asp Tyr Trp Gly Gln Gly Thr Thr
            100                 105                 110

Leu Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210>  18
<211>  220
<212>  PRT
<213>  Artificial sequence

<220>
<223>  Humanized antibody

<400>  18

Asp Ile Val Met Thr Gln Ser Pro Ser Ser Leu Ala Val Ser Val Gly
1               5                   10                  15

Glu Arg Val Thr Ile Ser Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Tyr Leu Arg Thr Phe Gly Gly Gly Thr Lys Leu Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210>  19
<211>  448
<212>  PRT
<213>  Artificial sequence

<220>
<223>  Humanized antibody

<400>  19

Gln Val Gln Leu Val Gln Ser Gly Ala Glu Val Val Lys Pro Gly Ala
1               5                   10                  15

Ser Val Lys Val Ser Cys Lys Ala Ser Gly Tyr Thr Phe Thr Gly Tyr
            20                  25                  30

Asp Leu His Trp Val Arg Gln Ala Pro Gly Gln Gly Leu Glu Trp Ile
        35                  40                  45

Gly Val Ile Ser Thr Tyr Tyr Gly Asp Ala Asn Tyr Asn Gln Lys Phe
    50                  55                  60

Gln Gly Arg Val Thr Met Thr Val Asp Thr Ser Ser Ser Thr Ala Tyr
65                  70                  75                  80

Met Glu Leu Ser Arg Leu Arg Ser Glu Asp Thr Ala Val Tyr Tyr Cys
                85                  90                  95

Thr Arg Leu Ala Glu Ser Tyr Phe Phe Asp Tyr Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser

225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  20
<211>  213
<212>  PRT
<213>  Artificial sequence

<220>
<223>  Humanized antibody

<400>  20

Glu Ile Val Leu Thr Gln Ser Pro Ala Thr Leu Ser Ala Ser Pro Gly
1               5                   10                  15

Glu Arg Val Thr Leu Thr Cys Arg Ala Ser Ser Ser Val Ser Tyr Leu
            20                  25                  30

His Trp Tyr Gln Gln Lys Pro Gly Gln Ala Pro Lys Pro Trp Ile Tyr
        35                  40                  45

Asp Thr Ser Asn Leu Ala Ser Gly Phe Pro Ala Arg Phe Ser Gly Asp
    50                  55                  60

Gly Ser Gly Thr Asp Tyr Thr Leu Thr Ile Ser Ser Leu Glu Ala Glu
65                  70                  75                  80

Asp Ala Ala Val Tyr Tyr Cys His Gln Arg Ser Ser Tyr Pro Phe Thr
                85                  90                  95

Phe Gly Ser Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  21
<211>  98
<212>  PRT
<213>  Homo sapiens

<400>  21

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Glu Met Asn Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg

<210>  22
<211>  101
<212>  PRT
<213>  Homo sapiens

<400>  22

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Leu Tyr Ser
            20                  25                  30

Ser Asn Asn Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Thr Pro
            100

<210>  23
<211>  98
<212>  PRT
<213>  Homo sapiens

<400>  23

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg

<210>  24
<211>  95
<212>  PRT
<213>  Homo sapiens

<400>  24

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro
                85                  90                  95

<210>  25
<211>  117
<212>  PRT
<213>  Artificial sequence

<220>
<223>  Humanized antibody

<400>  25

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Thr Pro Gly Lys Arg Leu Glu Trp Val
        35                  40                  45

Ala Tyr Ile Ser Ser Gly Gly Ser Thr Tyr Tyr Pro Asp Ser Val Lys
    50                  55                  60

Gly Arg Phe Thr Ile Ser Arg Glu Asn Ala Lys Asn Ile Leu Tyr Leu
65                  70                  75                  80

Gln Met Asn Ser Leu Arg Ala Glu Asp Thr Ala Val Tyr Tyr Cys Ala
                85                  90                  95

Arg Glu Gly Tyr Gly Thr Tyr Gly Asp Tyr Trp Gly Gln Gly Thr Thr
            100                 105                 110

Leu Thr Val Ser Ser
        115

<210>  26
<211>  117
<212>  PRT
<213>  Artificial sequence

<220>
<223>  Humanized antibody

<400>  26

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ala Tyr Ile Ser Ser Gly Gly Ser Thr Tyr Tyr Pro Asp Ser Val Lys
    50                  55                  60

Gly Arg Phe Thr Ile Ser Arg Asp Asn Ala Lys Asn Ser Leu Tyr Leu
65                  70                  75                  80

Gln Met Asn Ser Leu Arg Ala Glu Asp Thr Ala Val Tyr Tyr Cys Ala
                85                  90                  95

Arg Glu Gly Tyr Gly Thr Tyr Gly Asp Tyr Trp Gly Gln Gly Thr Thr
            100                 105                 110

Leu Thr Val Ser Ser
        115

<210>  27
<211>  117
<212>  PRT
<213>  Artificial sequence

<220>
<223>  Humanized antibody

<400>  27

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Tyr Ile Ser Ser Gly Gly Ser Thr Tyr Tyr Ala Asp Ser Val Lys
    50                  55                  60

Gly Arg Phe Thr Ile Ser Arg Asp Asn Ala Lys Asn Ser Leu Tyr Leu
65                  70                  75                  80

Gln Met Asn Ser Leu Arg Ala Glu Asp Thr Ala Val Tyr Tyr Cys Ala
                85                  90                  95

Arg Glu Gly Tyr Gly Thr Tyr Gly Asp Tyr Trp Gly Gln Gly Thr Thr
            100                 105                 110

Leu Thr Val Ser Ser
        115

<210>  28
<211>  113
<212>  PRT
<213>  Artificial sequence

<220>
<223>  Humanized antibody

<400>  28

Asp Ile Val Met Thr Gln Ser Pro Ser Ser Leu Ala Val Ser Val Gly
1               5                   10                  15

Glu Arg Val Thr Met Ser Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Gln
                85                  90                  95

Tyr Tyr Ser Tyr Leu Arg Thr Phe Gly Gly Gly Thr Lys Leu Glu Ile
            100                 105                 110

Lys

<210>  29
<211>  113
<212>  PRT
<213>  Artificial sequence

<220>
<223>  Humanized antibody

<400>  29

Asp Ile Val Met Thr Gln Ser Pro Ser Ser Leu Ala Val Ser Val Gly
1               5                   10                  15

Glu Arg Val Thr Ile Ser Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Tyr Leu Arg Thr Phe Gly Gly Gly Thr Lys Leu Glu Ile
            100                 105                 110

Lys

<210>  30
<211>  113
<212>  PRT
<213>  Artificial sequence

<220>
<223>  Humanized antibody

<400>  30

Asp Ile Val Met Thr Gln Ser Pro Ser Ser Leu Ala Val Ser Val Gly
1               5                   10                  15

Glu Arg Val Thr Ile Ser Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Tyr Leu Arg Thr Phe Gly Gly Gly Thr Lys Leu Glu Ile
            100                 105                 110

Lys

<210> 31
<211> 118
<212> PRT
<213> Artificial sequence

<220>
<223> Humanized antibody

<400> 31

Gln Val Gln Leu Val Gln Ser Gly Ala Glu Val Val Lys Pro Gly Ala
1               5                   10                  15

Ser Val Lys Ile Ser Cys Lys Ala Ser Gly Tyr Thr Phe Thr Gly Tyr
            20                  25                  30

Asp Leu His Trp Val Lys Gln Ala His Gly Gln Gly Leu Asp Trp Ile
        35                  40                  45

Gly Val Ile Ser Thr Tyr Tyr Gly Asp Ala Thr Tyr Asn Gln Lys Phe
    50                  55                  60

Gln Gly Arg Ala Thr Met Thr Val Asp Thr Ser Ser Ser Thr Ala Tyr
65                  70                  75                  80

Met Glu Leu Ser Arg Leu Thr Ser Glu Asp Thr Ala Val Tyr Tyr Cys
                85                  90                  95

Thr Arg Leu Ala Glu Ser Tyr Phe Phe Asp Tyr Trp Gly Gln Gly Thr
            100                 105                 110

Thr Leu Thr Val Ser Ser
        115

<210> 32
<211> 118
<212> PRT
<213> Artificial sequence

<220>
<223> Humanized antibody

<400> 32

Gln Val Gln Leu Val Gln Ser Gly Ala Glu Val Val Lys Pro Gly Ala
1               5                   10                  15

Ser Val Lys Val Ser Cys Lys Ala Ser Gly Tyr Thr Phe Thr Gly Tyr
            20                  25                  30

Asp Leu His Trp Val Arg Gln Ala Pro Gly Gln Gly Leu Glu Trp Ile
        35                  40                  45

Gly Val Ile Ser Thr Tyr Tyr Gly Asp Ala Asn Tyr Asn Gln Lys Phe
    50                  55                  60

Gln Gly Arg Val Thr Met Thr Val Asp Thr Ser Ser Ser Thr Ala Tyr
65                  70                  75                  80

Met Glu Leu Ser Arg Leu Arg Ser Glu Asp Thr Ala Val Tyr Tyr Cys
                85                  90                  95

Thr Arg Leu Ala Glu Ser Tyr Phe Phe Asp Tyr Trp Gly Gln Gly Thr
            100                 105                 110

Thr Leu Thr Val Ser Ser
        115

<210>  33
<211>  118
<212>  PRT
<213>  Artificial sequence

<220>
<223>  Humanized antibody

<400>  33

Gln Val Gln Leu Val Gln Ser Gly Ala Glu Val Val Lys Pro Gly Ala
1               5                   10                  15

Ser Val Lys Val Ser Cys Lys Ala Ser Gly Tyr Thr Phe Thr Gly Tyr
            20                  25                  30

Asp Leu His Trp Val Arg Gln Ala Pro Gly Gln Gly Leu Glu Trp Ile
        35                  40                  45

Gly Val Ile Ser Thr Tyr Tyr Gly Asp Ala Asn Tyr Asn Gln Lys Phe
    50                  55                  60

Gln Gly Arg Val Thr Met Thr Val Asp Thr Ser Ile Ser Thr Ala Tyr
65                  70                  75                  80

Met Glu Leu Ser Arg Leu Arg Ser Glu Asp Thr Ala Val Tyr Tyr Cys
                85                  90                  95

Thr Arg Leu Ala Glu Ser Tyr Phe Phe Asp Tyr Trp Gly Gln Gly Thr
            100                 105                 110

Thr Leu Thr Val Ser Ser
        115

<210>  34
<211>  106
<212>  PRT
<213>  Artificial sequence

<220>
<223>  Humanized antibody

<400>  34

Gln Ile Val Leu Thr Gln Ser Pro Ala Thr Leu Ser Ala Ser Pro Gly
1               5                   10                  15

Glu Arg Val Thr Met Thr Cys Arg Ala Ser Ser Ser Val Ser Tyr Ile
            20                  25                  30

His Trp Tyr Gln Gln Lys Pro Gly Gln Ala Pro Lys Pro Trp Ile Tyr
        35                  40                  45

Asp Thr Ser Asn Leu Ala Ser Gly Phe Pro Ala Arg Phe Ser Gly Asp
    50                  55                  60

Gly Ser Gly Thr Asp Tyr Ser Leu Ile Ile Ser Ser Met Glu Ala Glu
65                  70                  75                  80

Asp Ala Ala Thr Tyr Tyr Cys His Gln Arg Ser Ser Tyr Pro Phe Thr
                85                  90                  95

Phe Gly Ser Gly Thr Lys Leu Glu Ile Lys
            100                 105

<210>  35
<211>  106
<212>  PRT
<213>  Artificial sequence

<220>
<223>  Humanized antibody

<400>  35

Glu Ile Val Leu Thr Gln Ser Pro Ala Thr Leu Ser Ala Ser Pro Gly
1               5                   10                  15

Glu Arg Val Thr Leu Thr Cys Arg Ala Ser Ser Ser Val Ser Tyr Leu
            20                  25                  30

His Trp Tyr Gln Gln Lys Pro Gly Gln Ala Pro Lys Pro Trp Ile Tyr
        35                  40                  45

Asp Thr Ser Asn Leu Ala Ser Gly Phe Pro Ala Arg Phe Ser Gly Asp
    50                  55                  60

Gly Ser Gly Thr Asp Tyr Thr Leu Thr Ile Ser Ser Leu Glu Ala Glu
65                  70                  75                  80

Asp Ala Ala Val Tyr Tyr Cys His Gln Arg Ser Ser Tyr Pro Phe Thr
                85                  90                  95

Phe Gly Ser Gly Thr Lys Leu Glu Ile Lys
            100                 105

<210>  36
<211>  106
<212>  PRT
<213>  Artificial sequence

<220>
<223>  Humanized antibody

<400>  36

Glu Ile Val Leu Thr Gln Ser Pro Ala Thr Leu Ser Ala Ser Pro Gly
1               5                   10                  15

Glu Arg Val Thr Leu Thr Cys Arg Ala Ser Ser Ser Val Ser Tyr Leu
            20                  25                  30

His Trp Tyr Gln Gln Lys Pro Gly Gln Ala Pro Lys Pro Leu Ile Tyr
        35                  40                  45

Asp Thr Ser Asp Leu Ala Ser Gly Ile Pro Ala Arg Phe Ser Gly Asp
    50                  55                  60

Gly Ser Gly Thr Asp Tyr Thr Leu Thr Ile Ser Ser Leu Glu Ala Glu
65                  70                  75                  80

Asp Ala Ala Val Tyr Tyr Cys His Gln Arg Ser Ser Tyr Pro Phe Thr
                85                  90                  95

Phe Gly Ser Gly Thr Lys Leu Glu Ile Lys
            100                 105

<210>  37
<211>  121
<212>  PRT
<213>  Mus musculus

<400>  37

Asp Val Gln Leu Gln Glu Ser Gly Pro Gly Leu Val Lys Pro Ser Gln
1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asn Lys Leu Glu Trp
        35                  40                  45

Leu Gly Tyr Ile Ser Tyr Ser Gly Ser Thr Tyr Tyr Asn Pro Ser Leu
    50                  55                  60

Lys Ser Arg Ile Ser Leu Thr Arg Asp Thr Ser Lys Asn Gln Phe Phe
65                  70                  75                  80

Leu Gln Leu Asn Ser Val Thr Ser Glu Asp Thr Ala Thr Tyr Tyr Cys
                85                  90                  95

Ala Arg Trp Asp Tyr Arg Tyr Asp Gly Arg Gly Phe Asp Tyr Trp Gly
            100                 105                 110

Gln Gly Thr Thr Leu Thr Val Ser Ser
        115                 120

<210>  38
<211>  107
<212>  PRT
<213>  Mus musculus

<400>  38

Asp Ile Gln Met Thr Gln Ser Pro Ser Ser Leu Ser Ala Ser Leu Gly
1               5                   10                  15

Glu Arg Val Ser Leu Thr Cys Arg Ala Ser Gln Asp Ile Gly Ser Gly
            20                  25                  30

Leu Asn Trp Leu Gln Gln Glu Pro Asp Gly Thr Ile Lys Arg Leu Ile
        35                  40                  45

Tyr Ala Thr Ser Ser Leu Asp Ser Gly Val Pro Lys Arg Phe Ser Gly
    50                  55                  60

Ser Arg Ser Gly Ser Asp Tyr Ser Leu Thr Ile Ser Ser Leu Glu Ser
65                  70                  75                  80

Glu Asp Phe Val Asp Tyr Asn Cys Leu Gln Tyr Thr Ser Thr Pro Trp
                85                  90                  95

Thr Phe Gly Gly Gly Thr Lys Leu Glu Ile Lys
            100                 105

<210>  39
<211>  10
<212>  PRT
<213>  Mus musculus

<400>  39

Gly Tyr Ser Ile Thr Ser Asp Tyr Ala Trp
1               5                   10

<210>  40
<211>  16
<212>  PRT
<213>  Mus musculus

<400>  40

Tyr Ile Ser Tyr Ser Gly Ser Thr Tyr Tyr Asn Pro Ser Leu Lys Ser
1               5                   10                  15

<210>  41
<211>  12
<212>  PRT
<213>  Mus musculus

<400>  41

Trp Asp Tyr Arg Tyr Asp Gly Arg Gly Phe Asp Tyr
1               5                   10

<210>  42
<211>  11
<212>  PRT
<213>  Mus musculus

<400>  42

Arg Ala Ser Gln Asp Ile Gly Ser Gly Leu Asn
1               5                   10

<210>  43
<211>  7
<212>  PRT
<213>  Mus musculus

<400>  43

Ala Thr Ser Ser Leu Asp Ser
1               5

<210>  44
<211>  9
<212>  PRT
<213>  Mus musculus

<400>  44

Leu Gln Tyr Thr Ser Thr Pro Trp Thr
1               5

<210>  45
<211>  117
<212>  PRT
<213>  Mus musculus

<400>  45

Gln Val Gln Leu Gln Gln Ser Gly Ala Glu Leu Ala Lys Pro Gly Ala
1               5                   10                  15

Ser Val Lys Met Ser Cys Lys Ala Ser Gly Tyr Thr Phe Thr Thr Tyr
            20                  25                  30

Trp Ile His Trp Ile Lys Gln Arg Pro Gly Gln Gly Leu Glu Trp Ile
        35                  40                  45

Gly Tyr Ile Asn Pro Ser Thr Gly Tyr Thr Asp Tyr Asn Gln Asn Phe
    50                  55                  60

Lys Asp Lys Ala Thr Leu Thr Ala Asp Gln Tyr Ser Ser Thr Ala Tyr
65                  70                  75                  80

Met Gln Leu Ser Ser Leu Thr Phe Glu Asp Ser Ala Val Tyr Tyr Cys
                85                  90                  95

Ala Asn Tyr Tyr Gly Ser Thr Pro Tyr Tyr Trp Gly Arg Gly Thr Thr
            100                 105                 110

Leu Thr Val Ser Ser
        115

<210>  46
<211>  106
<212>  PRT
<213>  Mus musculus

<400>  46

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Thr Tyr Val
            20                  25                  30

His Trp Tyr Gln Gln Lys Ser Gly Ala Ser Pro Glu Arg Trp Ile Phe
        35                  40                  45

Asp Ala Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Thr Glu
65                  70                  75                  80

Asp Ala Ala Thr Tyr Tyr Cys Gln Gln Trp Ser Ser Asn Pro Leu Thr
                85                  90                  95

Phe Gly Ala Gly Thr Lys Leu Glu Leu Lys
            100                 105

<210>  47
<211>  10
<212>  PRT
<213>  Mus musculus

<400>  47

Gly Tyr Thr Phe Thr Thr Tyr Trp Ile His
1               5                   10

<210>  48
<211>  17
<212>  PRT
<213>  Mus musculus

<400>  48

Tyr Ile Asn Pro Ser Thr Gly Tyr Thr Asp Tyr Asn Gln Asn Phe Lys
1               5                   10                  15

Asp

<210>  49
<211>  8
<212>  PRT
<213>  Mus musculus

<400>  49

Tyr Tyr Gly Ser Thr Pro Tyr Tyr
1               5

<210>  50
<211>  10
<212>  PRT
<213>  Mus musculus

<400>  50

Ser Ala Ser Ser Ser Val Thr Tyr Val His

1               5                   10


<210>  51
<211>  7
<212>  PRT
<213>  Mus musculus

<400>  51

Asp Ala Ser Lys Leu Ala Ser
1               5


<210>  52
<211>  9
<212>  PRT
<213>  Mus musculus

<400>  52

Gln Gln Trp Ser Ser Asn Pro Leu Thr
1               5


<210>  53
<211>  403
<212>  PRT
<213>  Artificial sequence

<220>
<223>  Fusion protein

<400>  53

Met Ala Gln His Gly Ala Met Gly Ala Phe Arg Ala Leu Cys Gly Leu
1               5                   10                  15


Ala Leu Leu Cys Ala Leu Ser Leu Gly Gln Arg Pro Thr Gly Gly Pro
            20                  25                  30


Gly Cys Gly Pro Gly Arg Leu Leu Leu Gly Thr Gly Thr Asp Ala Arg
        35                  40                  45


Cys Cys Arg Val His Thr Thr Arg Cys Cys Arg Asp Tyr Pro Gly Glu
    50                  55                  60


Glu Cys Cys Ser Glu Trp Asp Cys Met Cys Val Gln Pro Glu Phe His
65                  70                  75                  80


Cys Gly Asp Pro Cys Cys Thr Thr Cys Arg His His Pro Cys Pro Pro
                85                  90                  95


Gly Gln Gly Val Gln Ser Gln Gly Lys Phe Ser Phe Gly Phe Gln Cys
            100                 105                 110

Ile Asp Cys Ala Ser Gly Thr Phe Ser Gly Gly His Glu Gly His Cys
        115                 120                 125

Lys Pro Trp Thr Asp Cys Thr Gln Phe Gly Phe Leu Thr Val Phe Pro
    130                 135                 140

Gly Asn Lys Thr His Asn Ala Val Cys Val Pro Gly Ser Pro Pro Ala
145                 150                 155                 160

Glu Pro Lys Leu Glu Asn Leu Tyr Phe Gln Gly Pro Arg Gly Pro Thr
                165                 170                 175

Ile Lys Pro Cys Pro Pro Cys Lys Cys Pro Ala Pro Asn Leu Leu Gly
            180                 185                 190

Gly Pro Ser Val Phe Ile Phe Pro Pro Lys Ile Lys Asp Val Leu Met
        195                 200                 205

Ile Ser Leu Ser Pro Ile Val Thr Cys Val Val Val Asp Val Ser Glu
    210                 215                 220

Asp Asp Pro Asp Val Gln Ile Ser Trp Phe Val Asn Asn Val Glu Val
225                 230                 235                 240

His Thr Ala Gln Thr Gln Thr His Arg Glu Asp Tyr Asn Ser Thr Leu
                245                 250                 255

Arg Val Val Ser Ala Leu Pro Ile Gln His Gln Asp Trp Met Ser Gly
            260                 265                 270

Lys Glu Phe Lys Cys Lys Val Asn Asn Lys Asp Leu Pro Ala Pro Ile
        275                 280                 285

Glu Arg Thr Ile Ser Lys Pro Lys Gly Ser Val Arg Ala Pro Gln Val
    290                 295                 300

Tyr Val Leu Pro Pro Pro Glu Glu Glu Met Thr Lys Lys Gln Val Thr
305                 310                 315                 320

Leu Thr Cys Met Val Thr Asp Phe Met Pro Glu Asp Ile Tyr Val Glu
                325                 330                 335

Trp Thr Asn Asn Gly Lys Thr Glu Leu Asn Tyr Lys Asn Thr Glu Pro
            340                 345                 350

Val Leu Asp Ser Asp Gly Ser Tyr Phe Met Tyr Ser Lys Leu Arg Val
        355                 360                 365

Glu Lys Lys Asn Trp Val Glu Arg Asn Ser Tyr Ser Cys Ser Val Val
    370                 375                 380

His Glu Gly Leu His Asn His His Thr Thr Lys Ser Phe Ser Arg Thr
385                 390                 395                 400

Pro Gly Lys

<210>  54
<211>  396
<212>  PRT
<213>  Homo sapiens

<400>  54

Met Ala Gln His Gly Ala Met Gly Ala Phe Arg Ala Leu Cys Gly Leu
1               5                   10                  15

Ala Leu Leu Cys Ala Leu Ser Leu Gly Gln Arg Pro Thr Gly Gly Pro
            20                  25                  30

Gly Cys Gly Pro Gly Arg Leu Leu Leu Gly Thr Gly Thr Asp Ala Arg
        35                  40                  45

Cys Cys Arg Val His Thr Thr Arg Cys Cys Arg Asp Tyr Pro Gly Glu
    50                  55                  60

Glu Cys Cys Ser Glu Trp Asp Cys Met Cys Val Gln Pro Glu Phe His
65                  70                  75                  80

Cys Gly Asp Pro Cys Cys Thr Thr Cys Arg His His Pro Cys Pro Pro
                85                  90                  95

Gly Gln Gly Val Gln Ser Gln Gly Lys Phe Ser Phe Gly Phe Gln Cys
            100                 105                 110

Ile Asp Cys Ala Ser Gly Thr Phe Ser Gly Gly His Glu Gly His Cys
        115                 120                 125

Lys Pro Trp Thr Asp Cys Thr Gln Phe Gly Phe Leu Thr Val Phe Pro
    130                 135                 140

Gly Asn Lys Thr His Asn Ala Val Cys Val Pro Gly Ser Pro Pro Ala
145                 150                 155                 160

Glu Pro Lys Leu Glu Asn Leu Tyr Phe Gln Gly Thr His Thr Cys Pro
                165                 170                 175

Pro Cys Pro Ala Pro Glu Leu Leu Gly Gly Pro Ser Val Phe Leu Phe
            180                 185                 190

Pro Pro Lys Pro Lys Asp Thr Leu Met Ile Ser Arg Thr Pro Glu Val
        195                 200                 205

Thr Cys Val Val Val Asp Val Ser His Glu Asp Pro Glu Val Lys Phe
    210                 215                 220

Asn Trp Tyr Val Asp Gly Val Glu Val His Asn Ala Lys Thr Lys Pro
225                 230                 235                 240

Arg Glu Glu Gln Tyr Asn Ser Thr Tyr Arg Val Val Ser Val Leu Thr
                245                 250                 255

Val Leu His Gln Asp Trp Leu Asn Gly Lys Glu Tyr Lys Cys Lys Val
            260                 265                 270

Ser Asn Lys Ala Leu Pro Ala Pro Ile Glu Lys Thr Ile Ser Lys Ala
        275                 280                 285

Lys Gly Gln Pro Arg Glu Pro Gln Val Tyr Thr Leu Pro Pro Ser Arg
    290                 295                 300

Asp Glu Leu Thr Lys Asn Gln Val Ser Leu Thr Cys Leu Val Lys Gly
305                 310                 315                 320

Phe Tyr Pro Ser Asp Ile Ala Val Glu Trp Glu Ser Asn Gly Gln Pro
                325                 330                 335

Glu Asn Asn Tyr Lys Thr Thr Pro Pro Val Leu Asp Ser Asp Gly Ser
            340                 345                 350

Phe Phe Leu Tyr Ser Lys Leu Thr Val Asp Lys Ser Arg Trp Gln Gln
        355                 360                 365

Gly Asn Val Phe Ser Cys Ser Val Met His Glu Ala Leu His Asn His
    370                 375                 380

Tyr Thr Gln Lys Ser Leu Ser Leu Ser Pro Gly Lys
385                 390                 395

<210>  55
<211>  159
<212>  PRT
<213>  Homo sapiens

<400>  55

Met Gly Val Leu Leu Thr Gln Arg Thr Leu Leu Ser Leu Val Leu Ala
1               5                   10                  15

Leu Leu Phe Pro Ser Met Ala Ser Met Gln Leu Glu Thr Ala Lys Glu
            20                  25                  30

Pro Cys Met Ala Lys Phe Gly Pro Leu Pro Ser Lys Trp Gln Met Ala
        35                  40                  45

Ser Ser Glu Pro Pro Cys Val Asn Lys Val Ser Asp Trp Lys Leu Glu
    50                  55                  60

Ile Leu Gln Asn Gly Leu Tyr Leu Ile Tyr Gly Gln Val Ala Pro Asn
65                  70                  75                  80

Ala Asn Tyr Asn Asp Val Ala Pro Phe Glu Val Arg Leu Tyr Lys Asn
                85                  90                  95

Lys Asp Met Ile Gln Thr Leu Thr Asn Lys Ser Lys Ile Gln Asn Val
            100                 105                 110

Gly Gly Thr Tyr Glu Leu His Val Gly Asp Thr Ile Asp Leu Ile Phe
        115                 120                 125

Asn Ser Glu His Gln Val Leu Lys Asn Asn Thr Tyr Trp Gly Ile Ile
    130                 135                 140

Leu Leu Ala Asn Pro Gln Phe Ile Ser His His His His His His
145                 150                 155


<210>  56
<211>  17
<212>  PRT
<213>  Artificial sequence

<220>
<223>  HCDR2

<400>  56

Val Ile Ser Thr Tyr Tyr Gly Asp Ala Asn Tyr Asn Gln Lys Phe Gln
1               5                   10                  15


Gly



<210>  57
<211>  10
<212>  PRT
<213>  Artificial sequence

<220>
<223>  LCDR3

<400>  57

Arg Ala Ser Ser Ser Val Ser Tyr Leu His
1               5                   10